# EUROPEAN PATENT APPLICATION

(11) **EP 2 821 414 A1**
(43) Date of publication of application: **07.01.2015**
(21) Application number: 14158299.9
(22) Date of filing: 18.06.2010
(51) Int. Cl.: C07K 14/435, A61K 8/64, A61K 38/10, A61K 38/17, A61P 31/04, A61Q 11/00, C07K 7/08

(54) **Kappa casein fragments inhibiting gingipains**

(30) Priority: 19.06.2009 AU 2009902841
(62) Divisional of application: 10788513.9
(71) Applicant: Oral Health Australia Pty Ltd, Carlton, Victoria 3053 (AU)
(72) Inventor: Reynolds, Eric Charles, Carlton, Victoria 3053 (AU); Dashper, Stuart Geoffrey, Carlton, Victoria 3053 (AU)
(74) Representative: Fleck, Barbara

(57) **Abstract**

The present invention relates to compounds, peptides, peptidomimetics and pharmaceutical compositions that inhibit protease activity and the use of these compounds, peptides, peptidomimetics and pharmaceutical compositions to treat or prevent a condition. In particular the condition may be periodontal disease. The compounds, peptides and peptidomimetics may be derived from various caseins, for example α-casein, β-casein and κ-casein. The protease activity which the compounds, peptides, peptidomimetics and pharmaceutical compositions of the invention inhibit includes that of the gingipains.

## Description

### Field of the invention

The present invention relates to compounds, peptides, peptidomimetics and pharmaceutical compositions that inhibit protease activity and the use of these compounds, peptides, peptidomimetics and pharmaceutical compositions to treat or prevent a condition. In particular the condition may be periodontal disease.

This application claims priority to Australian provisional application no. 2009902841, this application is incorporated herein in its entirety by reference.

### Background of the invention

Periodontal diseases are bacteria associated inflammatory diseases of the supporting tissues of the teeth and are a major public health problem. Nearly all of the human population is affected by periodontal diseases to some degree. A US Dental Health survey in 1989 reported that 85% of the studied population has periodontal diseases. The major form of periodontal disease is gingivitis which is associated with the nonspecific accumulation of dental plaque at the gingival margin. The more destructive form of periodontal disease (periodontitis) is associated with a subgingival infection by specific Gram-negative bacteria. The major bacterial pathogens implicated in this disease are known as the "red complex", which is composed of *Tannerella forsythia, Porphyromonas gingivalis,* and *Treponema denticola. P. gingivalis* is the main aetiological agent in chronic periodontitis.

The main virulence factors of *P. gingivalis* are thought to be its extracellular cysteine proteases, known collectively as the gingipains. Most common are RgpA and RgpB (the Arg-gingipains) and Kgp (the Lys-gingipain). The Arg-gingipains cleave at the carboxyl side of Arg residues and the Lys-gingipains cleave at the carboxyl side of Lys residues.

These cell-bound cysteine proteases are thought to be important for the degradation of proteins to provide peptides for growth as well as other intrinsic and extrinsic functions for survival and virulence. Several of these functions for survival and virulence may be bacterial adhesion to host tissue, hemagglutination, and the processing of bacterial cell-surface and secretory proteins. The catalytic domains of RgpA and Kgp can bind as a complex on the cell surface with a series of non-covalently bound sequence-related hemagglutinin/adhesin domains while RgpB has been shown to exist as not part of the protease adhesin complex and may consist of the catalytic domain only.

There have been several molecules both naturally derived and synthetic, which have been found to inhibit the gingipains. Natural inhibitors of gingipains have been found through the screening of bioactive products, while several synthetic inhibitors have been synthesized based on the structures of protease active sites and protease-inhibitors. For example, Arg- and Lys-gingipain inhibitors have been isolated from cranberry juice [specifically polyphenols and a high molecular weight, non-dialyzable constituent (NDM)], green tea catechins and garlic. These inhibitors suffer from one or more problems such as they are synthetic, costly to produce, have an unacceptable taste or are not approved for use in humans.

There exists a need for a better or alternative inhibitor of bacterial enzymes involved in the pathogenesis of various diseases, particularly periodontal disease.

Reference to any prior art in the specification is not, and should not be taken as, an acknowledgment or any form of suggestion that this prior art forms part of the common general knowledge in Australia or any other jurisdiction or that this prior art could reasonably be expected to be ascertained, understood and regarded as relevant by a person skilled in the art.

### Summary of the invention

According to the present invention there is provided a compound, peptide or peptidomimetic for inhibiting, reducing or preventing the activity of a bacterial enzyme, the compound, peptide or peptidomimetic comprising an amino acid sequence of a casein or fragment thereof. In one embodiment the enzyme may be an extracellular protease. In specific embodiments, the extracellular protease is a cysteine protease, such as a gingipain. In certain embodiments, the protease is RgpA, RgpB or Kgp.

In certain embodiments the compound, peptide or peptidomimetic comprises an amino acid sequence selected from the group consisting of:
Leu Pro Gln Glu Val Leu Asn Glu Asn Leu Leu Arg Phe (SEQ ID NO:1);
Tyr Gln Glu Pro Val Leu Gly Pro Val Arg Gly Pro Phe Pro Ile Ile Val (SEQ ID NO:2); 
Pro Pro Lys Lys Asn Gln Asp Lys Thr Glu Ile Pro Thr Ile Asn Thr Ile Ala Ser Gly Glu Pro
Thr Ser Thr Pro Thr Thr Glu (SEQ ID NO:3);
Tyr Gln Glu Pro Val Leu Gly Pro Val Arg Gly Pro Phe (SEQ ID NO:4);
Thr Glu Ile Pro Thr Ile Asn Thr Ile Ala Ser Gly Glu Pro Thr Ser Thr Pro Thr Thr Glu (SEQ ID NO:5).

In additional embodiments the compound, peptide or peptidomimetic comprises an amino acid sequence selected from the further group consisting of:
Leu Pro Gln Gly Val Leu Asn Glu Asn Leu Leu Arg Phe (SEQ ID NO:6);
Leu Ser Pro Glu Val Leu Asn Glu Asn Leu Leu Arg Phe (SEQ ID NO: 7);
Leu Ser Ser Glu Val Leu Asn Glu Asn Leu Leu Arg Phe (SEQ ID NO: 8);
Tyr Gln Glu Pro Val Leu Gly Pro Val Arg Gly Pro Phe Pro Ile Leu Val (SEQ ID NO: 9); 
Pro Pro Lys Lys Asn Gln Asp Lys Thr Glu Ile Pro Thr Ile Asn Thr Ile Ala Ser Gly Glu Pro
Thr Ser Thr Pro Thr Ile Glu (SEQ ID NO:10);
 
Pro Pro Lys Lys Asn Gln Asp Lys Thr Glu Ile Pro Thr Ile Asn Thr Ile Ala Ser Gly Glu Pro
Thr Ser Thr Pro Ile Thr Glu (SEQ ID NO:11);
 
Pro Pro Lys Lys Asn Gln Asp Lys Thr Glu Ile Pro Thr Ile Asn Thr Ile Ala Ser Gly Glu Pro
Thr Ser Thr(O-linked GalNAc) Pro Thr Thr Glu (SEQ ID NO:12);
 
Pro Pro Lys Lys Asn Gln Asp Lys Thr Glu Ile Pro Thr Ile Asn Thr Ile Ala Ser Gly Glu Pro
Thr(O-linked GalNAc) Ser Thr Pro Thr Thr Glu (SEQ ID NO:13);
 
Pro Pro Lys Lys Asn Gln Asp Lys Thr Glu Ile Pro Thr Ile Asn Thr Ile Ala Ser Gly Glu Pro
Thr Ser Thr Pro Thr Thr(O-linked GalNAc) Glu (SEQ ID NO:14);
 
Pro Pro Lys Lys Asn Gln Asp Lys Thr Glu Ile Pro Thr(O-linked GalNAc) Ile Asn Thr Ile
Ala Ser Gly Glu Pro Thr Ser Thr Pro Thr Thr Glu (SEQ ID NO:15);
 
Pro Pro Lys Lys Asn Gln Asp Lys Thr Glu Ile Pro Thr Ile Asn Thr Ile Ala Ser(P) Gly Glu
Pro Thr Ser Thr Pro Thr Thr Glu (SEQ ID NO:16);
 
Pro Pro Lys Lys Asn Gln Asp Lys Thr Glu Ile Pro Thr Ile Asn Thr Ile Val Ser Gly Glu Pro
Thr Ser Thr Pro Thr Thr Glu (SEQ ID NO:17);
 
Pro Pro Lys Lys Asn Gln Asp Lys Thr Glu Ile Pro Thr Ile Asn Thr Ile Ala Ser Val Glu Pro
Thr Ser Thr Pro Thr Thr Glu (SEQ ID NO:18);
 
Pro Pro Lys Lys Asp Gln Asp Lys Thr Glu Ile Pro Thr Ile Asn Thr Ile Ala Ser Gly Glu Pro
Thr Ser Thr Pro Thr Thr Glu (SEQ ID NO:19);
 
Pro Pro Lys Lys Asn Gln Asp Lys Thr Glu Ile Pro Thr Ile Asn Thr Ile Ala Ser Ala Glu Pro
Thr Ser Thr Pro Thr Thr Glu (SEQ ID NO:20);
 
Pro Pro Lys Lys Asp Gln Asp Lys Thr Glu Val Pro Ala Ile Asn Thr Ile Ala Ser Ala Glu
Pro Thr Ser Thr Pro Thr Thr Glu (SEQ ID NO: 21);
Thr Glu Ile Pro Thr Ile Asn Thr Ile Ala Ser Gly Glu Pro Thr Ser Thr Pro Thr Ile Glu (SEQ ID NO: 22);
Thr Glu Ile Pro Thr Ile Asn Thr Ile Ala Ser Gly Glu Pro Thr Ser Thr Pro Ile Thr Glu (SEQ ID NO: 23); 
Thr Glu Ile Pro Thr Ile Asn Thr Ile Ala Ser Gly Glu Pro Thr Ser Thr(O-linked GalNAc)
Pro Thr Thr Glu SEQ ID NO: 24);
 
Thr Glu Ile Pro Thr Ile Asn Thr Ile Ala Ser Gly Glu Pro Thr(O-linked GalNAc) Ser Thr
Pro Thr Thr Glu (SEQ ID NO: 25);
 
Thr Glu Ile Pro Thr Ile Asn Thr Ile Ala Ser Gly Glu Pro Thr Ser Thr Pro Thr Thr(O-linked
GalNAc) Glu (SEQ ID NO: 26);
 
Thr Glu Ile Pro Thr(O-linked GalNAc) Ile Asn Thr Ile Ala Ser Gly Glu Pro Thr Ser Thr
Pro Thr Thr Glu (SEQ ID NO: 27);
Thr Glu Ile Pro Thr Ile Asn Thr Ile Ala Ser(P) Gly Glu Pro Thr Ser Thr Pro Thr Thr Glu (SEQ ID NO: 28);
Thr Glu Ile Pro Thr Ile Asn Thr Ile Val Ser Gly Glu Pro Thr Ser Thr Pro Thr Thr Glu (SEQ ID NO: 29);
Thr Glu Ile Pro Thr Ile Asn Thr Ile Ala Ser Val Glu Pro Thr Ser Thr Pro Thr Thr Glu (SEQ ID NO: 30);
Thr Glu Ile Pro Thr Ile Asn Thr Ile Ala Ser Ala Glu Pro Thr Ser Thr Pro Thr Thr Glu (SEQ ID NO: 31), and
Thr Glu Val Pro Ala Ile Asn Thr Ile Ala Ser Ala Glu Pro Thr Ser Thr Pro Thr Thr Glu (SEQ ID NO: 32).

In other embodiments the compound, peptide or peptidomimetic comprises conservative substitutions in SEQ ID NOs: 1 to 32. These substitutions are described further below.

In certain embodiments, the peptide or peptidomimetic of the invention is at least 13 amino acids in length. In other embodiments, the peptide or peptidomimetic is 70 amino acid in length or less. In certain embodiments, the peptide or peptidomimetic is 15 to 61 amino acids in length. In other embodiments, the peptide or peptidomimetic is 20 to 30 amino acids in length.

In further embodiments, the compound, peptide or peptidomimetic is not phosphorylated or glycosylated. In another form, the compound, peptide or peptidomimetic is post-translationally modified. For example, the compound, peptide or peptidomimetic may be only phosphorylated or only glycosylated or both phosphorylated and glycosylated. One or more residues may be modified in this way.

In particular embodiments the compound, peptide or peptidomimetic consists of or consists essentially of an amino acid sequence selected from the group consisting of any one of SEQ ID NOs: 1 to 32 inclusive. In these embodiments, a compound, peptide or peptidomimetic that includes SEQ ID NOs: 1 to 32 as well as additional amino acid residues would "consist essentially of" SEQ ID NOs: 1 to 32 as long as it exhibits activity for inhibiting, reducing or preventing the activity of a bacterial enzyme, as may be determined in accordance with the assays described below. Similarly, a compound, peptide or peptidomimetic "consists essentially of" one of SEQ ID NO: 1 to 32 where it is shorter than the corresponding SEQ ID as long as it exhibits activity for inhibiting, reducing or preventing the activity of a bacterial enzyme, as may be determined in accordance with the assays described below. These embodiments thus do not include a full-length casein sequence.

In other embodiments, a compound, peptide or peptidomimetic of the invention comprises an amino acid sequence that is 60, 70, 80, 90, 91, 92, 93, 94, 95, 96, 97, 98, 99 or 100% identical to an amino acid sequence selected from the group consisting of SEQ ID NOs: 1 to 32. In other embodiments, the compound, peptide or peptidomimetic consists essentially of such an amino acid sequence. In some embodiments, the group consists of SEQ ID NOs: 1 to 5.

In certain embodiments there is provided a compound, peptide or peptidomimetic for inhibiting, reducing or preventing the activity of a bacterial enzyme, the compound, peptide or peptidomimetic comprising an amino acid sequence capable of forming a helical structure and which non-competitively inhibits, reduces or prevents the activity of a bacterial enzyme.

In other embodiments, the compound, peptide or peptidomimetic inhibits, reduces or prevents the enzyme from producing a product from a substrate such as by binding to the enzyme, substrate or both the enzyme and substrate when the enzyme interacts with a substrate.

In another embodiment, the invention provides a therapeutic peptide for use in inhibiting, reducing or preventing the activity of a *P. gingivalis* enzyme in the oral cavity of a patient in need, selected from the group consisting of SEQ ID NO: 1, SEQ ID NO: 2 and SEQ ID NO: 3 or glycosylated and phosphorylated variants and deletion and replacement mutants thereof. In some embodiments, the variants and mutants of SEQ ID NO: 1 are selected from the group consisting of SEQ ID NO: 6, SEQ ID NO: 7 and SEQ ID NO: 8. In some embodiments, the variants and mutants of SEQ ID NO: 2 are selected from the group consisting of SEQ ID NO: 4 and SEQ ID NO: 9. In some embodiments, the variants and mutants of SEQ ID NO: 3 are selected from the group consisting of SEQ ID NO: 5 and SEQ ID NOs :10 to 32.

In certain embodiments there is provided a composition for inhibiting a bacterial enzyme comprising a compound, peptide or peptidomimetic of the invention and a pharmaceutically acceptable carrier. In some embodiments, the composition further includes a divalent cation.

In one embodiment, there is provided a method for treating or preventing one or more conditions comprising administering to a subject in need an effective amount of compound, peptide, peptidomimetic or composition of the invention. In one embodiment, the compound, peptide, peptidomimetic or composition is administered directly to the gums of the subject. In one embodiment the compound, peptide or peptidomimetic may be a part of a composition applicable to the mouth such as dentifrice including toothpastes, toothpowders and liquid dentifrices, mouthwashes, troches, chewing gums, dental pastes, gingival massage creams, gargle tablets, dairy products and other foodstuffs. Examples of conditions or diseases suitable for treatment or prevention include periodontal disease and dental caries. Any disease or condition that is caused by or associated with the enzymatic activity of a gingipain may be suitable for treatment or prevention in accordance with the invention.

The subject in need of treatment or at risk of developing periodontal disease or dental caries is an animal. In some embodiments, the subject is a human, dog, cat, horse, sheep or cow.

In another embodiment there is provided a method of treating or alleviating a symptom of periodontal disease in a subject, comprising administering to the subject a compound, peptide, peptidomimetic or composition of the invention.

In accordance with some embodiments, there is provided a method of inhibiting a bacterial enzyme comprising contacting the enzyme with a composition comprising a compound, peptide or peptidomimetic that comprises, consists essentially of or consists of an amino acid sequence selected from the group consisting of any one of SEQ ID Nos: 1 to 5 and conservative substitutions therein, such as an amino acid sequence selected from the group consisting of SEQ ID Nos 1 to 32 and sequences at least 60% identical thereto. In specific embodiments, the amino acid sequence of the compound, peptide or peptidomimetic does not comprise a full-length casein sequence. In accordance with any of these embodiments, the bacterial enzyme may be a ginipain, such as a gingipain is from *Porphyromonas gingivalis.*

In accordance with any of these embodiments, the contacting step may comprise administering the composition to a subject (such as a human) in need thereof, such as a subject in need of treatment for or at risk of developing periodontal disease or dental caries. In accordance with any of these embodiments, the method may be effective to treat or alleviate a symptom of periodontal disease or dental caries.

In another embodiment a method of the invention further comprises administering an agent selected from the group consisting of anti-inflammatory agents, antibodies that bind to *P. gingivalis* or a protein expressed by *P. gingivalis,* antibiotics and antibiofilm agents. The antibiotic may be selected from the group consisting of amoxicillin, doxycycline and metronidazole. Anti-inflammatory agents include Nonsteroidal Anti-inflammatory Drugs (NSAIDs). Examples of NSAIDs include compounds than inhibit a cyclooxygenase. Specific examples of NSAIDs include aspirin, ibuprofen and naproxen. An example of an antibiofilm agent is an inhibitor of fumarate reductase, such as oxantel.

In another embodiment the invention provides a use of an effective amount of a compound, peptide, peptidomimetic or composition of the invention in the preparation of a medicament for the treatment or prevention of periodontal disease and/or the other conditions identified herein as suitable for treatment.

The present invention also provides a pharmaceutical composition for the treatment or prevention of periodontal disease (and/or the other conditions identified above as suitable for treatment) comprising an effective amount of a compound, peptide or peptidomimetic of the invention and a pharmaceutically acceptable carrier. The composition may further include an agent selected from the group consisting of anti-inflammatory agents, antibodies that bind to *P. gingivalis* or a protein from *P. gingivalis,* antibiotics and antibiofilm agents. The antibiotic may be selected from the group consisting of amoxicillin, doxycycline and metronidazole.

In accordance with some embodiments, there is provided a pharmaceutical composition comprising a compound, peptide or peptidomimetic that comprises, consists essentially of or consists of an amino acid sequence of a casein or fragment thereof that inhibits a bacterial enzyme, in an amount effective to inhibit a bacterial enzyme in a subject, such as an amino acid sequence selected from the group consisting of any one of SEQ ID Nos: 1 to 5 and conservative substitutions therein, or SEQ ID Nos 1 to 32 and sequences at least 60% identical thereto. In specific embodiments, the amino acid sequence of the compound, peptide or peptidomimetic does not comprise a full-length casein sequence.

In accordance with any of these embodiments, the composition may further comprise a divalent cation, such as zinc, and/or an agent selected from the group consisting of anti-inflammatory agents, antibiotics, antibiofilm agents and antibodies that bind to *Porphyromonas gingivalis* or a protein expressed by *Porphyromonas gingivalis.*

In accordance with any of these embodiments, the composition may be formulated for topical administration to the gums and/or may be provided in unit dosage form.

In another embodiment the invention provides a composition for the treatment or prevention of periodontal disease (and/or the other conditions identified above as suitable for treatment) comprising as an active ingredient a compound, peptide or peptidomimetic of the invention. The composition can further include a divalent cation.

In another embodiment the invention provides a pharmaceutical composition comprising an effective amount of a compound, peptide or peptidomimetic of the invention as a main ingredient. The composition may be used for example for the treatment or prevention of periodontal disease and/or the other conditions identified above as suitable for treatment. In some embodiments, the composition further comprises a divalent cation.

In another embodiment the invention provides a compound, peptide or peptidomimetic of the invention for use in the treatment or prevention of periodontal disease and/or the other conditions identified above as suitable for treatment.

In another embodiment the invention provides a composition comprising a compound, peptide or peptidomimetic of the invention for use in the treatment or prevention of periodontal disease. In some embodiments, the composition further comprises a divalent cation.

In a further aspect, the present invention provides a kit of parts including (a) a compound, peptide, peptidomimetic or composition of the invention and (b) a pharmaceutically acceptable carrier.

In certain embodiments, the divalent cation is selected from the group consisting of Zn²⁺, Ca²⁺, Cu²⁺, Ni²⁺, Co²⁺, Fe²⁺, Sn²⁺, and Mn²⁺. In addition, the divalent cation may be in association with fluoride such as SnF⁺ and CuF⁺. In some embodiments, the divalent cation is Ca²⁺ or Zn²⁺.

In certain embodiments, the ratio of the divalent cation to the compound, peptide or peptidomimetic is in the range of 1.0:2.0 to 1.0:10.0, such as in the range of 1.0:4.0.

As used herein, except where the context requires otherwise, the term "comprise" and variations of the term, such as "comprising", "comprises" and "comprised", are not intended to exclude further additives, components, integers or steps.

### Brief description of the drawings

**Figure 1****:** *P. gingivalis* ATCC 33277 whole cell Arg-specific proteolytic activity with synthetic α_{S1}-casein(11-23) (■), β-casein(193-205) (□), β-casein(193-209) ( 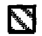 ) and κ-casein(109-137) ( 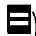 ) peptide. Assays were performed with 2 separate bacterial cultures and three technical replicates. The average number of cells in the assay is 4.0E+08 cfu/mL.
**Figure 2****:** *P. gingivalis* ATCC 33277 whole cell Lys-specific proteolytic activity with synthetic α_{S1}-casein(11-23) (■), β-casein(193-209) ( 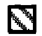 ) and κ-casein(109-137) ( 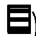 ) peptide. Assays were performed with 2 separate bacterial cultures and three technical replicates. The average number of cells in the assay is 4.0E+08 cfu/mL.
**Figure 3****:** Purified protease complex Arg- ( 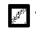 ) and Lys-specific ( 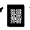 ) proteolytic activity with 100 µM synthetic casein-derived peptides. Assays were performed with six technical replicates.
**Figure 4****:** Purified RgpB proteolytic activity with several concentrations of κ-casein(109-137). The concentration of RgpB is 0.23 µg/µL.

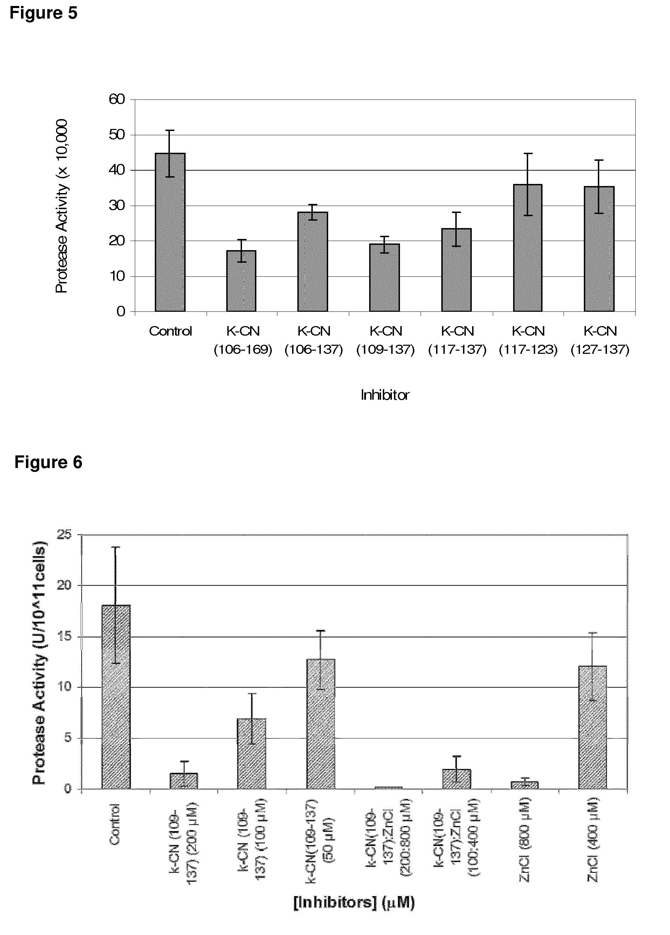

**Figure 5****:** Arg- and Lys-specific proteinase activity of *P. gingivalis* ATCC 33277 whole cells measured using fluorescent BSA substrate (DQTM BSA) and 500 µM casein peptides. κ-casein(106-169) was naturally derived while the other κ-casein peptides were synthetic. The error bars were calculated as a standard deviation of three technical replicates and two biological replicates. All peptides were significantly different (p < 0.05) from the control values. κ-casein(117-123) and κ-casein(127-137) are not significantly different (p < 0.05) to each other but are significantly different to the rest of the peptides. κ-casein(106-169) was significantly different from κ-casein(106-137) but not κ-casein(109-137) or κ-casein(117-137). κ-casein(106-137) was significantly different from all peptides except κ-casein(117-137). κ-casein(109-137) was significantly different from all peptides except κ-casein(106-169).
**Figure 6****:** *P. gingivalis* ATCC 33277 whole cell Arg-specific proteolytic activity with synthetic κ-casein(109-137) peptide and ZnCl₂ at 1 mM cysteine concentration in the assay. Assays were performed with three separate bacterial cultures and four technical replicates.
**Figure 7****:** *P. gingivalis* ATCC 33277 whole cell Lys-specific proteolytic activity with synthetic κ-casein(109-137) peptide and ZnCl₂ at 1 mM cysteine concentration in the assay. Assays were performed with two separate bacterial cultures and three technical replicates.
**Figure 8****:** Purified protease complex Arg- ( 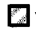 ) and Lys-specific ( 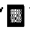 ) proteolytic activity with synthetic κ-casein(109-137) peptide and ZnCl₂ at 1 mM cysteine concentration in the assay. Assays were performed with six technical replicates.
**Figure 9****:** *P. gingivalis* ATCC 33277 whole cell Arg-specific proteolytic activity with synthetic α_{S1}-casein(11-23) peptide ( 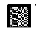 ), β-casein (193-209) ( 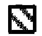 ), and ZnCl₂ at 1 mM cysteine concentration in the assay. Assays were performed with two separate bacterial cultures and three technical replicates.
**Figure 10****:** *P. gingivalis* ATCC 33277 whole cell Lys-specific proteolytic activity with synthetic α_{S1}-casein(11-23) peptide ( 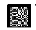 ), β-casein (193-209) ( 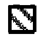 ), and ZnCl₂ at 1 mM cysteine concentration in the assay. Assays were performed with two separate bacterial cultures and three technical replicates.
**Figure 11****:** Lineweaver-Burk plots of inhibition of purified RgpB by κ-casein(109-137) peptide at concentrations of 0 µM (-×-), 25 µM (-◆-), 50 µM (-■-), 75 µM (-▲-) and 100 µM (-×-) with the substrate BApNA at concentrations of 0.15, 0.25, and 1.0 mM.
**Figure 12****:** A secondary plot for the estimation of inhibition constant (Kᵢ) for inhibition of RgpB by κ-casein(109-137) peptide. Lines associated with each set of points represent linear regression analysis of the respective data sets. Kᵢ was estimated as the negative intercept of the regression line.
**Figure 13****:** BLAST sequence alignment of κ-casein(109-137) and human serine/cysteine proteinase inhibitor clade G member 1 splice variant 3 (Q5UG15), Plasma serine protease C1 inhibitor (P05155), and Putative serine proteinase inhibitor (KU family)(A3LQ30).
**Figure 14****:** BLAST sequence alignment of β-casein(193-209) with human serine protease inhibitor Kazal-type 5 short isoform (Q3LX95), human serine protease inhibitor Kazal-type 5 (Q9NQ38), human Elafin (Elastase-specific inhibitor) (P19957), and human P13 protein (Peptidase inhibitor 3, skin-derived (SKALP), isoform CRA_a)(Q6FG74).
**Figure 15****:** BLAST sequence alignment of α_{S1}-casein(11-23) with ATP-dependent Clp protease ATP-binding subunit clpX (P50866), Serine protease (Q1NE66), and ATP-dependent zinc metalloproteinase (Q7VHT4).
**Figure 16****:** Proposed uncompetitive inhibitor model with E = Arg- or Lys-gingipain, S = BApNA or GPKNA substrate, I = κ-casein (109-137) peptide and P = *p*-nitroanilide product.

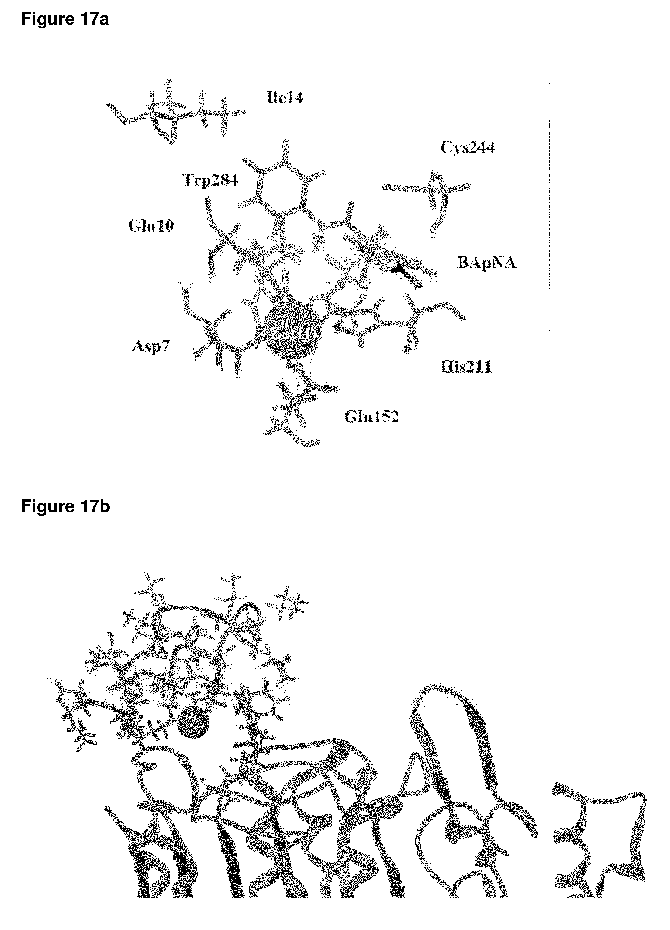

**Figure 17****:** a) Residues of RgpB active site involved in interacting with κ-casein (109-137), Zn(II) and the substrate (BApNA). The electrostatic interactions between His211 and Glu152 of RgpB, Asp115 (Asp7) and Glu118 (Glu10) of κ-casein (109-137) with Zn(II) are highlighted. The hydrophobic interactions between Ile122 (Ile14) of the peptide with the BApNA substrate are also highlighted. The Trp284 and Cys244 of RgpB form interactions with the Arg residue and the amide bond of the substrate respectively. b) Proposed molecular model of κ-casein(109-137) binding to the RgpB:BApNA complex in the presence of Zn(II).

### Detailed description of the embodiments

It will be understood that the invention disclosed and defined in this specification extends to all alternative combinations of two or more of the individual features mentioned or evident from the text or drawings. All of these different combinations constitute various alternative aspects of the invention.

Compounds, peptide or peptidomimetics that exhibit protease inhibitory can be used in oral care products, functional foods, and pharmaceuticals. The present invention identified new milk casein peptides that have been characterized by their *P. gingivalis* extracellular protease inhibitory activity. These peptides may be produced synthetically or obtained from enzymatic digestion of milk caseins. The peptides may be obtained from milk casein from various species, including humans, cows, goats and sheep.

Bovine milk caseins are a natural source of protein which are known to be relatively resistant to further proteolytic breakdown. They have been detected in the distal portion of the small intestine and blood of humans after ingestion of cow's milk.

These peptides have several advantages including, but not limited to, that they can be derived from a natural source, have no appreciable taste and can mask the taste of divalent cations such as zinc when used in combination. The peptides include amino acid sequences selected from the group consisting of:
α_{S1}-casein(11-23) - (SEQ ID NO:1)
β-casein(193-209) - (SEQ ID NO:2)
κ-casein(109-137) - (SEQ ID NO:3)
β-casein(193-205) - (SEQ ID NO:4)
κ-casein(117-137) - (SEQ ID NO:5).

The invention also includes functional fragments of the amino acid sequences of SEQ ID NO: 1 to 5. A functional fragment is an amino acid sequence that is shorter than the amino acid sequences corresponding to SEQ ID NO:1 to 5 but still retains the function of the corresponding amino acid sequences to SEQ ID NO: 1 to 5. A functional fragment can be easily determined by shortening the amino acid sequence, for example using an exopeptidase, or by sythesizing amino acid sequences of shorter length, and then testing for any protease inhibitory activity such as by the methods illustrated in the examples below.

Also within the scope of the invention are variants of the amino acid sequences of SEQ ID NO: 1 to 5 which correspond to fragments of orthologous and paralogous proteins to the bovine caseins from which SEQ ID NOS 1 to 5 are derived.

Sequences are "paralogous" if they were separated by a gene duplication event: if a gene in an organism is duplicated to occupy two different positions in the same genome, then the two copies are paralogous.

Sequences are "orthologous" if they were separated by a speciation event: when a species diverges into two separate species, the divergent copies of a single gene in the resulting species are said to be orthologous.

Another group of variants within the scope of the invention are the amino acid sequences of SEQ ID NO: 1 to 5 that contain post-translational modifications. Particular post-translational modifications are phosphorylation and glycosylation. To illustrate these modifications, a number of known genetic variants of bovine casein are known as follows:
A. α_{S1}-Caseins
   1. α_{S1}-Casein X^{a}-9P (genetic variants- A, B, C, D, E, F, G and H)
   2. α_{S1}-Casein fragments
B. α_{S2} -Caseins
   1. α_{S2} -Casein X^{a}-15P (genetic variants- A, B, C, D)
   2. α_{S2} -Casein X^{a}-9P (f51-207) (genetic variants- A, C)
   3. α_{S2} -Casein X^{a}-5P (f65-203) (genetic variants- A, C)
C. β-Caseins
   1. β-Casein X^{a}-5P (genetic variants- A¹, A², A³, B, C, D, E, F, G, H¹, H² and I)
   2. β-Casein X^{a}-1P (f29-209) (genetic variants- A¹, A², A³, B, C, E, F, G, H¹, H² and I)
   3. β-Casein X^{a}-(f106-209) (genetic variants- A², A³, B, F, G, H²)
   4. β-Casein X^{a}- (f 108-209) (genetic variants- A², B, F, G and H²)
   5. β-Casein X^{a}-4P (f1-28) (genetic variants- A², D and H¹)
   6. β-Casein X^{a}-5P (f1-105) (genetic variants- A¹, A², B, C, D, E, F, G, H¹, H² and I)
   7. β-Casein X^{a}-5P (f1-107) (genetic variants- A¹, A², A³, B, C, D, E, F, G, H¹, H² and I)
   8. β-Casein X^{a}-1P (f29-105) (genetic variants- A¹, A², B, C, E, F, G, H¹, H² and I)
   9. β-Casein X^{a}-1P (f29-107) (genetic variants- A¹, A², A³, B, C, E, F, G, H¹, H² and I)
D. κ-Caseins
   1. κ-Casein X^{a}-2P (genetic variants- A, B, C, E, F¹, F², G¹, G², H, I and J)
   2. κ-Casein X^{a}-2P (f106-169) (genetic variants- A, B, E, F¹, F², G¹, G² and J)
   3. κ-Casein X^{a}-2P (f117-169) (genetic variants- A, B, E, F¹, F², G¹, G² and J)
   ^{a} X indicates a genetic variant, where the genetic variant may be any one of the variants stated in the following brackets.

The above nomenclature describes known variants of casein, for example, β-casein B-1P (f29-209) indicates that the protein is part of the β-family of casein, is the B genetic variant, contains an amino acid residue that can be phosphorylated and is a fragment of the β-casein protein from residue 29 to residue 209. It is known which amino acids are susceptible to phosphorylation in these proteins. The nomenclature and further description of casein variants is described in Farrell et al. Nomenclature of Proteins of Cow's Milk - Sixth Revision. Journal of Dairy Science (2004)87:1641-1674. Accordingly, the fragments of these sequences that correspond to the amino acid sequences of SEQ ID NO: 1 to 5 are variants, and form part of the invention disclosed.

As it is believed that it is the physical nature of the peptides rather than the specific sequence of the peptide which results in their protease inhibitory activity, so called conservative substitutions may be made in the peptide sequence with no substantial loss of activity. Without limiting the invention, in some embodiments, up to 25% of the amino acids of the peptide or peptidomimetic are conservatively substituted. It is intended that such conservative substitutions which do not result in a substantial loss of activity are encompassed in the present invention. Whilst the concept of conservative substitution referred to above is well understood by the person skilled in the art, for the sake of clarity conservative substitutions are those set out below.
Gly, Ala, Val, Ile, Leu, Met;
Asp, Glu, Ser;
Asn, Gln;
Ser, Thr;
Lys, Arg, His;
Phe, Tyr, Trp; and
Pro, Nα-alkalamino acids.

Any substitution of one member of a polar, charged (+), charged (-) or aliphatic group of amino acids, as known to a person skilled in the art, by another member of the same group can be a conservative substitution.

To exemplify these additional embodiments (without limiting the invention), the following sequences are provided:
α_{S1}-casein(11-23),
Leu Pro Gln Glu Val Leu Asn Glu Asn Leu Leu Arg Phe (SEQ ID NO:1) (*Bos taurus* (cow))
Leu Pro Gln Gly Val Leu Asn Glu Asn Leu Leu Arg Phe (SEQ ID NO: 6) *(Bubalus bubalis* (Domestic water buffalo))
Leu Ser Pro Glu Val Leu Asn Glu Asn Leu Leu Arg Phe (SEQ ID NO: 7) *(Capra hircus* (Goat))
Leu Ser Ser Glu Val Leu Asn Glu Asn Leu Leu Arg Phe (SEQ ID NO: 8) *(Ovis aries* (Sheep))
β-casein(193-209),
Tyr Gln Glu Pro Val Leu Gly Pro Val Arg Gly Pro Phe Pro Ile Ile Val (SEQ ID NO:2) (*Bos taurus* (cow))
Tyr Gln Glu Pro Val Leu Gly Pro Val Arg Gly Pro Phe Pro Ile Leu Val (SEQ ID NO: 9) *(Capra hircus* (Goat), *Ovis aries* (Sheep))
β-casein(193-205),
Tyr Gln Glu Pro Val Leu Gly Pro Val Arg Gly Pro Phe (SEQ ID NO:4) (*Bos taurus* (cow))
κ-casein(109-137), 
Pro Pro Lys Lys Asn Gln Asp Lys Thr Glu Ile Pro Thr Ile Asn Thr Ile Ala Ser Gly Glu Pro
Thr Ser Thr Pro Thr Thr Glu (SEQ ID NO:3) (Bos taurus (cow))
 
Pro Pro Lys Lys Asn Gln Asp Lys Thr Glu Ile Pro Thr Ile Asn Thr Ile Ala Ser Gly Glu Pro
Thr Ser Thr Pro Thr Ile Glu (SEQ ID NO: 10) (Syncerus caffer nanus (Forest Buffalo))
 
Pro Pro Lys Lys Asn Gln Asp Lys Thr Glu Ile Pro Thr Ile Asn Thr Ile Ala Ser Gly Glu Pro
Thr Ser Thr Pro Ile Thr Glu (SEQ ID NO: 11) (Bos indicus (Zebu))
 
Pro Pro Lys Lys Asn Gln Asp Lys Thr Glu Ile Pro Thr Ile Asn Thr Ile Ala Ser Gly Glu Pro
Thr Ser Thr(O-linked GalNAc) Pro Thr Thr Glu (SEQ ID NO: 12) (Bos taurus (cow))
 
Pro Pro Lys Lys Asn Gln Asp Lys Thr Glu Ile Pro Thr Ile Asn Thr Ile Ala Ser Gly Glu Pro
Thr(O-linked GalNAc) Ser Thr Pro Thr Thr Glu (SEQ ID NO: 13) (Bos taurus (cow))
 
Pro Pro Lys Lys Asn Gln Asp Lys Thr Glu Ile Pro Thr Ile Asn Thr Ile Ala Ser Gly Glu Pro
Thr Ser Thr Pro Thr Thr(O-linked GalNAc) Glu (SEQ ID NO: 14) (Bos taurus (cow))
 
Pro Pro Lys Lys Asn Gln Asp Lys Thr Glu Ile Pro Thr(O-linked GalNAc) Ile Asn Thr Ile
Ala Ser Gly Glu Pro Thr Ser Thr Pro Thr Thr Glu (SEQ ID NO: 15) (Bos taurus (cow))
 
Pro Pro Lys Lys Asn Gln Asp Lys Thr Glu Ile Pro Thr Ile Asn Thr Ile Ala Ser(P) Gly Glu
Pro Thr Ser Thr Pro Thr Thr Glu (SEQ ID NO: 16) (Bos taurus (cow))
 
Pro Pro Lys Lys Asn Gln Asp Lys Thr Glu Ile Pro Thr Ile Asn Thr Ile Val Ser Gly Glu Pro
Thr Ser Thr Pro Thr Thr Glu (SEQ ID NO: 17) (Bubalus bubalis (Domestic water
buffalo))
 
Pro Pro Lys Lys Asn Gln Asp Lys Thr Glu Ile Pro Thr Ile Asn Thr Ile Ala Ser Val Glu Pro
Thr Ser Thr Pro Thr Thr Glu (SEQ ID NO: 18) (Bubalus bubalis (Domestic water
buffalo))
 
Pro Pro Lys Lys Asp Gln Asp Lys Thr Glu Ile Pro Thr Ile Asn Thr Ile Ala Ser Gly Glu Pro
Thr Ser Thr Pro Thr Thr Glu (SEQ ID NO: 19) (Oreamnos americanus (Mountain goat))
 
Pro Pro Lys Lys Asn Gln Asp Lys Thr Glu Ile Pro Thr Ile Asn Thr Ile Ala Ser Ala Glu Pro
Thr Ser Thr Pro Thr Thr Glu (SEQ ID NO: 20) (Oreamnos americanus (Mountain goat))
 
Pro Pro Lys Lys Asp Gln Asp Lys Thr Glu Val Pro Ala Ile Asn Thr Ile Ala Ser Ala Glu
Pro Thr Ser Thr Pro Thr Thr Glu (SEQ ID NO: 21) (Capra hircus (Goat), Ovis aries
(Sheep))
κ-casein(117-137),
Thr Glu Ile Pro Thr Ile Asn Thr Ile Ala Ser Gly Glu Pro Thr Ser Thr Pro Thr Thr Glu (SEQ ID NO:5) (Bos taurus (cow))
Thr Glu Ile Pro Thr Ile Asn Thr Ile Ala Ser Gly Glu Pro Thr Ser Thr Pro Thr Ile Glu (SEQ ID NO: 22) *(Syncerus caffer nanus* (Forest Buffalo))
Thr Glu Ile Pro Thr Ile Asn Thr Ile Ala Ser Gly Glu Pro Thr Ser Thr Pro Ile Thr Glu (SEQ ID NO: 23) (*Bos indicus* (Zebu)) 
Thr Glu Ile Pro Thr Ile Asn Thr Ile Ala Ser Gly Glu Pro Thr Ser Thr(O-linked GalNAc)
Pro Thr Thr Glu (SEQ ID NO: 24) (Bos taurus (cow))
 
Thr Glu Ile Pro Thr Ile Asn Thr Ile Ala Ser Gly Glu Pro Thr(O-linked GalNAc) Ser Thr
Pro Thr Thr Glu (SEQ ID NO: 25) (Bos taurus (cow))
 
Thr Glu Ile Pro Thr Ile Asn Thr Ile Ala Ser Gly Glu Pro Thr Ser Thr Pro Thr Thr(O-linked
GalNAc) Glu (SEQ ID NO: 26) (Bos taurus (cow))
 
Thr Glu Ile Pro Thr(O-linked GalNAc) Ile Asn Thr Ile Ala Ser Gly Glu Pro Thr Ser Thr
Pro Thr Thr Glu (SEQ ID NO: 27) (Bos taurus (cow))
Thr Glu Ile Pro Thr Ile Asn Thr Ile Ala Ser(P) Gly Glu Pro Thr Ser Thr Pro Thr Thr Glu (SEQ ID NO: 28) (*Bos taurus* (cow))
Thr Glu Ile Pro Thr Ile Asn Thr Ile Val Ser Gly Glu Pro Thr Ser Thr Pro Thr Thr Glu (SEQ ID NO: 29)(*Bubalus bubalis* (Domestic water buffalo))
Thr Glu Ile Pro Thr Ile Asn Thr Ile Ala Ser Val Glu Pro Thr Ser Thr Pro Thr Thr Glu (SEQ ID NO: 30) *(Bubalus bubalis* (Domestic water buffalo))
Thr Glu Ile Pro Thr Ile Asn Thr Ile Ala Ser Ala Glu Pro Thr Ser Thr Pro Thr Thr Glu (SEQ ID NO: 31) *(Oreamnos americanus* (Mountain goat))
Thr Glu Val Pro Ala Ile Asn Thr Ile Ala Ser Ala Glu Pro Thr Ser Thr Pro Thr Thr Glu (SEQ ID NO: 32) *(Capra hircus* (Goat), *Ovis aries* (Sheep))
(P) designates that the preceding amino acid is phosphorylated i.e. Ser(P) is a serine amino acid that is phosphorylated.

(O-linked GalNAc) designates that the preceding amino acid has an N-Acetylgalactosamine (GalNAc) attached to the hydroxy oxygen of the amino acid side chain i.e. Thr(O-linked GalNAc) is a threonine amino acid that has an N-Acetylgalactosamine attached to the hydroxy oxygen of its side chain. Other glycans may be attached to the GalNAc increasing the glycan chain length to include, disaccharides for example Gal(β1-3)GalNAc, trisaccharides for example NeuAc(α2-3)Gal(β1-3)GalNAc or Gal(β1-3)[NeuAc(α2-6)]GalNAc and tetrasaccharides, for example NeuAc(α2-3)Gal(β1-3)[NeuAc(α2-6)]GalNac.

The invention also provides compounds, peptides or peptidomimetics that comprise an amino acid sequence selected from the group consisting of: DEPTXPTTE (where X = TQ or ST), QEPVX₁GPVRGPX₂PIIX ₃I (where X₁ = L, N or K X₂ = F, Y or C and X₃ = L, H or V) and EVLNENLLRF.

The invention in there embodiments provides a compound, peptide or peptidomimetic which consists of or consists essentially of an amino acid sequence selected from the group consisting of any one of SEQ ID NOs: 1 to 32 inclusive but does not include a full-length casein sequence.

It will be understood by a person skilled in the art that one or more amino acid deletions to the amino acid sequence defined by any one of SEQ ID Nos: 1 to 32 may be made without losing the capacity of the compound, peptide or peptidomimetic to inhibit, reduce or prevent protease activity. In certain embodiments, up to 25% of a peptide or peptidomimetic including SEQ ID NOs: 1 to 32 may be deleted, however the resulting peptide or peptidomimetic must retain the capacity to inhibit, reduce or prevent protease activity. Experiments, including those described herein, can be performed to determined whether a compound, peptide or peptidomimetic that has an amino acid sequence that differs to any one of SEQ ID Nos: 1 to 32 by one or more amino acid deletions can still inhibit, reduce or prevent protease activity.

The compound, peptide, peptidomimetic or composition of the invention may be administered directly to the gums of the subject in need of treatment or prevention of periodontal disease. In some embodiments, the composition of the invention is topically administered, however it will be appreciated by a person skilled in the art that a compound, peptide, peptidomimetic or composition may also be administered parenterally, e. g, by injection intravenously, intraperitoneally, intramuscularly, intrathecally or subcutaneously.

Alternatively, the compound, peptide, peptidomimetic of the invention may be formulated as a composition for oral administration (including sublingual and buccal), pulmonary administration (intranasal and inhalation), transdermal administration, or rectal administration.

A subject in need of treatment may be one which exhibits subclinical or clinical symptoms of periodontal disease. Subclinical or clinical manifestations of periodontal disease include acute or chronic inflammation of the gingiva. The hallmarks of acute inflammation may be present including an increased movement of plasma and leukocytes from the blood into the injured tissues. Clinical signs of acute infection of the gingiva may also be present including rubor (redness), calor (increased heat), tumor (swelling), dolor (pain), and functio laesa (loss of function). Chronic inflammation may be characterised by leukocyte cell (monocytes, macrophages, lymphocytes, plasma cells) infiltration. Tissue and bone loss may be observed. A subject in need of treatment may also be characterised by having an increased level of *P. gingivalis* bacteria present at a periodontal site, above a normal range observed in individuals without periodontal disease.

The route of administration may depend on a number of factors including the nature of the antagonist or composition to be administered and the severity of the subject's condition. It is understood that the frequency of administration of a compound, peptide, peptidomimetic of the invention and the amount of compound, peptide, peptidomimetic of the invention administered may be varied from subject to subject depending on, amongst other things, the stage of periodontal disease initiation or progression in the subject. The frequency of administration may be determined by a clinician.

It is also contemplated that any disease, condition or syndrome that is a consequence of or associated with protease activity of a gingipain or related protease (for example, other cysteine proteases), may be prevented or treated by a compound, peptide, peptidomimetic or composition of the invention. Furthermore, other diseases, conditions or syndromes that are a consequence of or associated with periodontal disease may also be treated or the risk of developing these diseases, conditions or syndromes may be reduced. For example, periodontal disease may increase the risk of an individual developing cardiovascular disease. This increase risk of developing cardiovascular disease may be reduced by treating periodontal disease by administering a compound, peptide, peptidomimetic or composition of the invention to an individual with periodontal disease.

A 'peptidomimetic' is a synthetic chemical compound that has substantially the same structure and/or functional characteristics of a peptide of the invention, the latter being described further herein. Typically, a peptidomimetic has the same or similar structure as a peptide of the invention, for example the same or similar sequence of a casein or fragment thereof. A peptidomimetic generally contains at least one residue that is not naturally synthesised. Non-natural components of peptidomimetic compounds may be according to one or more of: a) residue linkage groups other than the natural amide bond ('peptide bond') linkages; b) non-natural residues in place of naturally occurring amino acid residues; or c) residues which induce secondary structural mimicry, i.e., to induce or stabilize a secondary structure, e.g., a beta turn, gamma turn, beta sheet, alpha helix conformation, and the like.

Peptidomimetics can be synthesized using a variety of procedures and methodologies described in the scientific and patent literatures, e.g., Organic Syntheses Collective Volumes, Gilman et al. (Eds) John Wiley & Sons, Inc., NY, al-Obeidi (1998) Mol. Biotechnol. 9:205-223; Hruby (1997) Curr. Opin. Chem. Biol. 1:114-119; Ostergaard (1997) Mol. Divers. 3:17-27; Ostresh (1996) Methods Enzymol. 267:220-234.

The compounds, peptides or peptidomimetics of the invention can be administered in the form of a pharmaceutical composition. These compositions may be manufactured under GMP conditions or in some embodiments by means of conventional mixing, dissolving, granulating, dragee-making, levigating, emulsifying, encapsulating, entrapping or lyophilizing processes.

Pharmaceutical compositions may be formulated using one or more physiologically acceptable carriers, diluents, excipients or auxiliaries. The ingredients may facilitate processing peptides or peptidomimetics into preparations which can be used pharmaceutically.

Administration for treatment can be parenteral, intravenous, oral, subcutaneous, intraarterial, intracranial, intrathecal, intraperitoneal, topical, intranasal or intramuscular.

Pharmaceutical compositions for parenteral administration are generally sterile and substantially isotonic. Physiologically compatible buffers such as Hank's solution, Ringer's solution, or physiological saline or acetate buffer may be used. The solution may also contain suspending, stabilizing and/or dispersing agents. The peptides or peptidomimetics may be provided in powder form to be dissolved in solvent such as sterile pyrogen-free water, before use.

"Percent (%) amino acid sequence identity" or " percent (%) identical" with respect to a peptide or polypeptide sequence, i.e. a peptide of the invention defined herein, is defined as the percentage of amino acid residues in a candidate sequence that are identical with the amino acid residues in the specific peptide or polypeptide sequence, i.e. a peptide of the invention, after aligning the sequences and introducing gaps, if necessary, to achieve the maximum percent sequence identity, and not considering any conservative substitutions as part of the sequence identity.

Those skilled in the art can determine appropriate parameters for measuring alignment, including any algorithms (non-limiting examples described below) needed to achieve maximal alignment over the full-length of the sequences being compared. When amino acid sequences are aligned, the percent amino acid sequence identity of a given amino acid sequence A to, with, or against a given amino acid sequence B (which can alternatively be phrased as a given amino acid sequence A that has or comprises a certain percent amino acid sequence identity to, with, or against a given amino acid sequence B) can be calculated as: percent amino acid sequence identity = X/Y100, where X is the number of amino acid residues scored as identical matches by the sequence alignment program's or algorithm's alignment of A and B and Y is the total number of amino acid residues in B. If the length of amino acid sequence A is not equal to the length of amino acid sequence B, the percent amino acid sequence identity of A to B will not equal the percent amino acid sequence identity of B to A.

In calculating percent identity, typically exact matches are counted. The determination of percent identity between two sequences can be accomplished using a mathematical algorithm. A nonlimiting example of a mathematical algorithm utilized for the comparison of two sequences is the algorithm of Karlin and Altschul (1990) Proc. Natl. Acad. Sci. USA 87:2264, modified as in Karlin and Altschul (1993) Proc. Natl. Acad. Sci. USA 90:5873-5877. Such an algorithm is incorporated into the BLASTN and BLASTX programs of Altschul et al. (1990) J. Mol. Biol. 215:403. To obtain gapped alignments for comparison purposes, Gapped BLAST (in BLAST 2.0) can be utilized as described in Altschul et al. (1997) Nucleic Acids Res. 25:3389. Alternatively, PSI-Blast can be used to perform an iterated search that detects distant relationships between molecules. See Altschul et al. (1997) supra. When utilizing BLAST, Gapped BLAST, and PSI-Blast programs, the default parameters of the respective programs (e.g., BLASTX and BLASTN) can be used. Alignment may also be performed manually by inspection. Another non- limiting example of a mathematical algorithm utilized for the comparison of sequences is the ClustalW algorithm (Higgins et al. (1994) Nucleic Acids Res. 22:4673-4680). ClustalW compares sequences and aligns the entirety of the amino acid or DNA sequence, and thus can provide data about the sequence conservation of the entire amino acid sequence. The ClustalW algorithm is used in several commercially available DNA/amino acid analysis software packages, such as the ALIGNX module of the Vector NTI Program Suite (Invitrogen Corporation, Carlsbad, CA). After alignment of amino acid sequences with ClustalW, the percent amino acid identity can be assessed. A non-limiting examples of a software program useful for analysis of ClustalW alignments is GENEDOC™ or JalView (http://www.jalview.org/). GENEDOC™ allows assessment of amino acid (or DNA) similarity and identity between multiple proteins. Another non-limiting example of a mathematical algorithm utilized for the comparison of sequences is the algorithm of Myers and Miller (1988) CABIOS 4:11-17. Such an algorithm is incorporated into the ALIGN program (version 2.0), which is part of the GCG Wisconsin Genetics Software Package, Version 10 (available from Accelrys, Inc., 9685 Scranton Rd., San Diego, CA, USA). When utilizing the ALIGN program for comparing amino acid sequences, a PAM 120 weight residue table, a gap length penalty of 12, and a gap penalty of 4 can be used.

Although the invention finds application in humans, the invention is also useful for veterinary purposes. The invention is useful for the treatment or prevention of a disease or condition, as described herein, in domestic animals such as cattle, sheep, horses and poultry; companion animals such as cats and dogs; and zoo animals.

An oral composition of this invention which contains the above-mentioned pharmaceutical composition can be prepared and used in various forms applicable to the mouth such as dentifrice including toothpastes, toothpowders and liquid dentifrices, mouthwashes, troches, chewing gums, dental pastes, gingival massage creams, gargle tablets, dairy products and other foodstuffs. An oral composition according to this invention may further include additional well known ingredients depending on the type and form of a particular oral composition.

Optionally, the composition may further include one or more antibiotics that are toxic to or inhibit the growth of Gram negative anaerobic bacteria. Potentially any bacteriostatic or bactericidal antibiotic may be used in a composition of the invention. Suitable antibiotics include amoxicillin, doxycycline or metronidazole.

In certain forms of the invention the oral composition may be substantially liquid in character, such as a mouthwash or rinse. In such a preparation the vehicle is typically a water-alcohol mixture desirably including a humectant as described below. Generally, the weight ratio of water to alcohol is in the range of from about 1:1 to about 20:1. The total amount of water-alcohol mixture in this type of preparation is typically in the range of from about 70 to about 99.9% by weight of the preparation. The alcohol is typically ethanol or isopropanol. In certain embodiments, the alcohol is ethanol.

The pH of such liquid and other preparations of the invention is generally in the range of from about 5 to about 9 and typically from about 5.0 to 7.0. The pH can be controlled with acid (e.g. citric acid or benzoic acid) or base (e.g. sodium hydroxide) or buffered (as with sodium citrate, benzoate, carbonate, or bicarbonate, disodium hydrogen phosphate, sodium dihydrogen phosphate, etc).

In other desirable forms of this invention, the composition may be substantially solid or pasty in character, such as toothpowder, a dental tablet or a toothpaste (dental cream) or gel dentifrice. The vehicle of such solid or pasty oral preparations generally contains dentally acceptable polishing material.

In toothpaste, the liquid vehicle may comprise water and humectant typically in an amount ranging from about 10% to about 80% by weight of the preparation. Glycerine, propylene glycol, sorbitol and polypropylene glycol exemplify suitable humectants/carriers. Also advantageous are liquid mixtures of water, glycerine and sorbitol. In clear gels where the refractive index is an important consideration, about 2.5 - 30% w/w of water, 0 to about 70% w/w of glycerine and about 20-80% w/w of sorbitol are typically employed.

Toothpaste, creams and gels typically contain a natural or synthetic thickener or gelling agent in proportions of about 0.1 to about 10, such as about 0.5 to about 5% w/w. A suitable thickener is synthetic hectorite, a synthetic colloidal magnesium alkali metal silicate complex clay available for example as Laponite (e.g. CP, SP 2002, D) marketed by Laporte Industries Limited. Laponite D is, approximately by weight 58.00% SiO2, 25.40% MgO, 3.05% Na2O, 0.98% Li2O, and some water and trace metals. Its true specific gravity is 2.53 and it has an apparent bulk density of 1.0 g/ml at 8% moisture.

Other suitable thickeners include Irish moss, iota carrageenan, gum tragacanth, starch, polyvinylpyrrolidone, hydroxyethylpropylcellulose, hydroxybutyl methyl cellulose, hydroxypropyl methyl cellulose, hydroxyethyl cellulose (e.g. available as Natrosol), sodium carboxymethyl cellulose, and colloidal silica such as finely ground Syloid (e.g. 244). Solubilizing agents may also be included such as humectant polyols such propylene glycol, dipropylene glycol and hexylene glycol, cellosolves such as methyl cellosolve and ethyl cellosolve, vegetable oils and waxes containing at least about 12 carbons in a straight chain such as olive oil, castor oil and petrolatum and esters such as amyl acetate, ethyl acetate and benzyl benzoate.

It will be understood that, as is conventional, the oral preparations will usually be sold or otherwise distributed in suitable labelled packages. Thus, a bottle of mouth rinse will have a label describing it, in substance, as a mouth rinse or mouthwash and having directions for its use; and a toothpaste, cream or gel will usually be in a collapsible tube, typically aluminium, lined lead or plastic, or other squeeze, pump or pressurized dispenser for metering out the contents, having a label describing it, in substance, as a toothpaste, gel or dental cream.

Organic surface-active agents may be used in the compositions of the present invention to achieve increased therapeutic or prophylactic action, assist in achieving thorough and complete dispersion of the active agent throughout the oral cavity, and render the instant compositions more cosmetically acceptable. The organic surface-active material may be anionic, non-ionic or ampholytic in nature and in some embodiments does not interact with the active agent. It is typical to employ as the surface-active agent a detersive material which imparts to the composition detersive and foaming properties. Suitable examples of anionic surfactants are water-soluble salts of higher fatty acid monoglyceride monosulfates, such as the sodium salt of the monosulfated monoglyceride of hydrogenated coconut oil fatty acids, higher alkyl sulfates such as sodium lauryl sulfate, alkyl aryl sulfonates such as sodium dodecyl benzene sulfonate, higher alkylsulfo-acetates, higher fatty acid esters of 1,2-dihydroxy propane sulfonate, and the substantially saturated higher aliphatic acyl amides of lower aliphatic amino carboxylic acid compounds, such as those having 12 to 16 carbons in the fatty acid, alkyl or acyl radicals, and the like. Examples of the last mentioned amides are N-lauroyl sarcosine, and the sodium, potassium, and ethanolamine salts of N-lauroyl, N-myristoyl, or N-palmitoyl sarcosine which should be substantially free from soap or similar higher fatty acid material. The use of these sarconite compounds in the oral compositions of the present invention is particularly advantageous since these materials exhibit a prolonged marked effect in the inhibition of acid formation in the oral cavity due to carbohydrates breakdown in addition to exerting some reduction in the solubility of tooth enamel in acid solutions. Examples of water-soluble non-ionic surfactants suitable for use are condensation products of ethylene oxide with various reactive hydrogencontaining compounds reactive therewith having long hydrophobic chains (e.g. aliphatic chains of about 12 to 20 carbon atoms), which condensation products ("ethoxamers") contain hydrophilic polyoxyethylene moieties, such as condensation products of poly (ethylene oxide) with fatty acids, fatty alcohols, fatty amides, polyhydric alcohols (e.g. sorbitan monostearate) and polypropyleneoxide (e.g. Pluronic materials).

The surface active agent is typically present in amount of about 0.1-5% by weight. It is noteworthy, that the surface active agent may assist in the dissolving of the active agent of the invention and thereby diminish the amount of solubilizing humectant needed.

Various other materials may be incorporated in the oral preparations of this invention such as whitening agents, preservatives, silicones, chlorophyll compounds and/or ammoniated material such as urea, diammonium phosphate, and mixtures thereof. These adjuvants, where present, are incorporated in the preparations in amounts which do not substantially adversely affect the properties and characteristics desired.

Any suitable flavouring or sweetening material may also be employed. Examples of suitable flavouring constituents are flavouring oils, e.g. oil of spearmint, peppermint, wintergreen, sassafras, clove, sage, eucalyptus, marjoram, cinnamon, lemon, and orange, and methyl salicylate. Suitable sweetening agents include sucrose, lactose, maltose, sorbitol, xylitol, sodium cyclamate, perillartine, AMP (aspartyl phenyl alanine, methyl ester), saccharine, and the like. Suitably, flavour and sweetening agents may each or together comprise from about 0.1 % to 5% more of the preparation.

The compound, peptide or peptidomimetic of composition of the invention can also be incorporated in lozenges, or in chewing gum or other products, e.g. by stirring into a warm gum base or coating the outer surface of a gum base, illustrative of which are jelutong, rubber latex, vinylite resins, etc., desirably with conventional plasticizers or softeners, sugar or other sweeteners or such as glucose, sorbitol and the like.

In a further aspect, the present invention provides a kit of parts including (a) a compound, peptide, peptidomimetic or composition and (b) a pharmaceutically acceptable carrier. Desirably, the kit further includes instructions for their use for the treatment or prevention of periodontal disease in a patient in need of such treatment.

Compositions intended for oral use may be prepared according to any method known in the art for the manufacture of pharmaceutical compositions and such compositions may contain one or more agents selected from the group consisting of sweetening agents, flavouring agents, colouring agents and preserving agents in order to provide pharmaceutically elegant and palatable preparations.

Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin or olive oil.

Aqueous suspensions contain the active materials in admixture with excipients suitable for the manufacture of aqueous suspensions. Such excipients are suspending agents, for example sodium carboxymethylcellulose, methylcellulose, hydropropyl methylcellulose, sodium alginate, polyvinylpyrrolidone, gum tragacanth and gum acacia; dispersing or wetting agents may be a naturally-occurring phosphatide, for example, lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate.

The aqueous suspensions may also contain one or more preservatives, for example benzoates, such as ethyl, or n-propyl p-hydroxybenzoate, one or more colouring agents, one or more flavouring agents, and one or more sweetening agents, such as sucrose or saccharin.

Oily suspensions may be formulated by suspending the active ingredients in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents such as those set forth above, and flavouring agents may be added to provide palatable oral preparations. These compositions may be preserved by the addition of an anti-oxidant such as ascorbic acid.

In particular, further aspects of the invention are set forth in the following clauses:
1. A compound, peptide or peptidomimetic for inhibiting, reducing or preventing the activity of a bacterial enzyme, wherein the compound, peptide or peptidomimetic consists essentially of an amino acid sequence selected from the group consisting of SEQ ID NO: 1, SEQ ID NO: 2, SEQ ID NO:3, SEQ ID NO: 4 and SEQ ID NO: 5 and conservative substitutions therein.
2. A compound, peptide or peptidomimetic according to clause 1, wherein the amino acid sequence is selected from the group consisting of SEQ ID NO: 1, SEQ ID NO: 2, SEQ ID NO:3, SEQ ID NO: 4 and SEQ ID NO: 5.
3. A compound, peptide or peptidomimetic according to clause 1, wherein the amino acid sequence is SEQ ID NO: 5.
4. A compound, peptide or peptidomimetic for inhibiting, reducing or preventing the activity of a bacterial enzyme consisting of an amino acid sequence that is at least 60% identical to any one of SEQ ID NOs: 1 to 32.
5. A compound, peptide or peptidomimetic according to any one of clauses 1 to 4, wherein the bacterial enzyme is a gingipain.
6. A compound, peptide or peptidomimetic according to clause 5, wherein the gingipain is from *Porphyromonas gingivalis.*
7. A pharmaceutical composition for inhibiting a bacterial enzyme comprising a compound, peptide or peptidomimetic comprising an amino acid sequence of casein or a fragment thereof and a pharmaceutically acceptable carrier.
8. A pharmaceutical composition according to clause 7, wherein the amino acid sequence consists essentially of any one or more of SEQ ID NO: 1, SEQ ID NO: 2, SEQ ID NO: 3, SEQ ID NO: 4 or SEQ ID NO: 5 and conservative substitutions therein.
9. A pharmaceutical composition according to clause 7 or 8, further comprising a divalent cation.
10. A pharmaceutical composition according to clause 9, wherein the divalent cation is zinc.
11. A pharmaceutical composition for use in the treatment of periodontal disease comprising a compound, peptide or peptidomimetic according to any one of clause s 1 to 6 and a pharmaceutically acceptable carrier.
12. A pharmaceutical composition according to clause 11, further comprising a divalent cation.
13. A pharmaceutical composition according to clause 12, wherein the divalent cation is zinc.
14. Use of a compound, peptide or peptidomimetic according to any one of clause s 1 to 6 in the preparation of a medicament for the treatment of periodontal disease.
15. A method of preventing or treating periodontal disease comprising administering to a subject in need an effective amount of a compound, peptide, peptidomimetic according to any one of clauses 1 to 6.
16. A method of inhibiting a bacterial enzyme comprising contacting the enzyme with a composition comprising a compound, peptide or peptidomimetic that comprises an amino acid sequence selected from the group consisting of any one of SEQ ID NOs: 1 to 32 and conservative substitutions therein.

To describe the invention in more detail, the following examples are described to illustrate some aspects and embodiments of the invention.

### Materials and Methods

### Enzyme Digestion and Purification of Peptides

Caseinate-HCl (Bonlac Foods, Melbourne Australia) was dissolved by slow addition with constant stirring to deionised water at 50°C, pH 8.0 to give a final concentration of 21.5 g/L. Once the caseinate had dissolved, the temperature was lowered to 37°C and the pH adjusted to 6.3 by the slow addition of 1 M HCl to avoid precipitation of casein. To begin the hydrolysis Rennet (90% Chymosin EC 3.4.23.4, 145 IMCU/ml, Single Strength, Chr. Hanson) was added to a final concentration of 1.2 IMCU/g casein and the solution stirred at 37°C for 1 h. The pH of the solution was maintained at 6.3 ± 0.2 by the addition of 1 M HCl and 1 M NaOH. Hydrolysis was stopped by the addition of trichloroacetic acid to a final concentration of 4% and the precipitated proteins were pelleted by centrifugation (5,000 g, 15 min, 4°C). The supernatant containing the caseinomacropeptide (CMP) was concentrated by diafiltration using a 3000 Da cutoff membrane (S10Y3, Amicon). This material was then lyophilized. The preparation was fractionated using a Superose 12 column (Amersham Biosciences, USA) connected to an ÄKTA Explorer system (Amersham Pharmacia Biotech, USA), and eluted using 50 mM Tris-HCl pH 8.0 at a flow rate of 0.5 ml/min. The eluent was monitored at a wavelength of 214 nm and 280 nm. Fractions were collected every 2 minutes. All samples were lyophilised in a Christ Freeze Dryer (Osterode am Harz, Germany) and stored at -70°C. The fractions were further fractionated by reversed phase HPLC using a C₁₈ column and eluted with 90% acetonitrile/0.1% v/v TFA. The eluant was monitored with a primary wavelength of 214 nm using an Agilent 1100S diode array and multiple wavelength detector (Agilent Tech., Palo Alto, California). All fractions collected were lyophilised and stored at -70°C. The identity of each fraction was confirmed by mass spectrometric analysis.

Non-glycosylated κ-casein(106-169) was obtained by chymosin digestion as previously described (Malkoski et al., 2001). κ-casein(106-137) was obtained by hydrolysis of non-glycosylated κ-casein(106-169) dissolved in 50 mM ammonium acetate pH 4.0 buffer with endoprotease-Glu-C from *Staphylococcus aureus* strain V8 (Roche, Penzberg Germany) at 37°C (E:S; 1:200) for 24 h. The hydrolysis was terminated by increasing the pH to 6.0 by addition of 2 M NaOH and peptides were separated using analytical (C₁₈) RP-HPLC. Collected fractions were analysed and peptides identified using MS/MS analysis.

### MALDI TOF/TOF MS

Peptide samples were co-crystallized (1:1 vol/vol) on a MTP 384 target ground steel plate with saturated 2,5-dihydroxybenzoic acid (DHB) matrix in standard buffer (50% acetonitrile, 0.1% TFA). The samples were analysed on an Ultraflex MALDI TOF/TOF Mass Spectrometer (Bruker, Bremen, Germany). Analysis was performed using Bruker Daltonics flexAnalysis 2.4 and Bruker Daltonics BioTools 3.0 software with fragmentation spectra matched to a casein database installed on a local MASCOT server.

### Solid Phase Peptide Synthesis

The α_{S1}-casein(11-23), β-casein(193-205), β-casein(193-209), κ-casein(106-137), κ-casein(109-137), κ-casein(117-137), κ-casein(117-123) and κ-casein(127-137) peptides were prepared using standard Fmoc-chemistry protocols on a Liberty™ Microwave peptide synthesizer (CEM Corporation, North Carolina). Peptide synthesis proceeded from the amide terminus on a Wang resin bound with the respective C-termini amino acids. Peptides were purified by reversed phase HPLC using a C₁₈ column.

### Electrospray MS

Fractions collected from the RP-HPLC were analysed using an Esquire-LC MS/MS system (Bruker Daltonics) operating in the electrospray mass spectrometry mode. Sample injection was conducted at 340 µL/h, with nitrogen flow of 5 L/min and drying gas temperature of 300°C.

### Bacterial Strains and Growth Conditions

Glycerol or freeze-dried cultures of *Porphyromonas gingivalis* W50 and ATCC 33277 cells were grown anaerobically at 37°C on Horse Blood Agar (HBA; Oxoid). *P. gingivalis* cells were maintained by passages and only passage 3-7 were used to inoculate 20 mL and 200 mL Brain Heart Infusion broth (37 g/L), supplemented with hemin (5 mg/L) and cysteine (0.5 g/L) and for ATCC 33277, vitamin K₃ (menadione) (5 mg/L) (BHI). Growth was determined by measurement of culture optical density (OD) at a wavelength of 650 nm. Gram stains of the cultures were carried out to check for any contamination. The P. *gingivalis* cells were harvested during exponential growth phase by centrifugation (8000 g, 20 min, 4°C) and washed once with TC150 buffer (50 mM Tris-HCl, 150 mM NaCl, 5 mM CaCl₂, pH 8.0) containing 0.5 g/L cysteine. The washed cells were resuspended in 2 mL of TC150 buffer (with 0.5 g/L cysteine), and kept at 4 °C to be used immediately in the proteolytic assays.

### Purification of P. gingivalis RgpA-Kgp proteinase adhesin complexes and RgpB.

*P. gingivalis* W50 cells were grown to late exponential phase in 2 L batch cultures and the RgpA-Kgp proteinase adhesin complexes were purified from Triton X114 extracts using Arg-sepharose affinity chromatography based on the procedure described previously (Pathirana et al., 2006). RgpB was purified from *P. gingivalis* strain HG66 using previously published procedures (Chen et al., 2002; Pike et al., 1994).

### Determination of P. gingivalis Arg- and Lys-specific proteolytic activity using chromogenic substrates

Bacterial protease inhibitory activity was determined using an assay developed for whole cell Arg- and Lys-specific proteolytic activity of *P. gingivalis* that was modified and adapted to be performed using 96-well plates with reduced incubation volumes (O'Brien-Simpson et al., 1998; O'Brien-Simpson et al., 2001). Arg- and Lys-specific proteolytic activity was determined using synthetic chromogenic substrates; N-α-benzoyl-Arg-p-nitroanilide (L-BApNA) and N-(p-Tosyl)-Gly-Pro-Lys 4-nitroanilide acetate salt (GPK-NA) (Sigma Aldrich). The Arg- and Lys-specific reaction buffer contained 2 mM L-BApNA or GPK-NA, respectively dissolved in 30% v/v isopropanol, 0.93 mM L-cysteine, 400 mM Tris-HCl pH 8.0, and 100 mM NaCl. The casein peptides were diluted with TC150 buffer depending on the concentration required. 10 µL of 10 mM L-cysteine pH 8.0 and either *P. gingivalis* whole cell suspension, purified RgpA-Kgp proteinase-adhesin complexes (0.01 µg/µL) or purified RgpB (0.00116 µg/µL) was added to a final volume of 100 µL. Samples were then incubated for 15 min at 37°C before adding the Arg- or Lys-specific reaction buffer (total volume of 200 µL). Activity was determined by measuring the absorbance at 405 nm at 10 s intervals for ∼20 min at 37°C, pH 8.0 using a PerkinElmer 1420 Multilabel Counter VICTOR3™ (Waltham, MA). A stock of 8 mM ZnCl₂ solution was prepared in deionised H₂O and used to dissolve the lyophilised casein peptides at the concentrations required for the specific assays. Control samples were identical to the test samples however they lacked any protease inhibitory substances i.e. peptides and/or zinc.

Following incubation, assay contents were analysed by RP-HPLC using an analytical (C₁₈) column and a linear gradient of 0-100% buffer B to determine if there was casein peptide degradation during the assay. Eluted fractions were analysed by ESI-MS and MS/MS analysis.

### Determination of P. gingivalis proteolytic activity with fluorescently-labelled bovine serum albumin.

Bacterial protease inhibitory activity was also determined using DQ™ Green BSA (Molecular Probes, Eugene, OR) with modifications from previously published procedures (Grenier et al., 2002; Yoshioka et al., 2003). *P. gingivalis* ATCC 33277 whole cells, harvested during exponential growth (O.D₆₅₀ₙₘ = 0.6) were used for the assay with 5.6 x 10⁹ cfu/mL per well. The assay mixture contained *P. gingivalis* cells (100 µL culture), TC150 and inhibitors (final volume 80 µl), and DQ BSA (20 µL; 200 µg/mL). 1 mM *N*α-*p*-tosyl-I-lysine chloromethylketone (TLCK) treated cells were used as controls. TLCK is known to inhibit both Rgp and Kgp activity (Fletcher et al., 1994, Pike et al., 1994). The assay mixtures were incubated in the dark for 2 h at 37°C prior to measuring the fluorescence (Em 535 nm, Ex 485 nm) using a fluorometer (PerkinElmer 1420 Multilabel Counter VICTOR3™). The fluorescence value obtained from the negative control (TLCK-treated cells) was subtracted from all values. All assays were performed in triplicate with 2-3 biological replicates unless stated otherwise.

### Fractional Inhibitory Concentration Index

The fractional inhibitory concentration index (FIC index) is a scale that defines the interaction of two or more inhibitors or antimicrobials; synergistically, additively, or antagonistically (Berenbaum, 1978). The FIC index is calculated as; FIC index = [(A+B)/ A] + [(A +B)/B], where A represents the effects of κ-casein (109-137) and B the effect of Zinc Chloride, and A+B the effect of the combination of both. An FIC index of >1 indicates an antagonistic effect, FIC index = 1 indicates an additive effect, and an FIC index of <1 indicates a synergistic effect.

### Determination of Type of Inhibition and Inhibition Constants (Kᵢ)

Initial reaction rates were obtained as OD₄₀₅/sec at substrate (BApNA) concentrations of 0.15, 0.25, and 1 mM. Inhibitor (peptide) concentrations ranged from 0, 25, 50, 75, and 100 µM The data was graphed as a Lineweaver-Burk (double reciprocal) plot and the slopes were determined by first-order linear regression. The Lineweaver-Burk plots can be used to determine the Kₘ (enzyme affinity) and Vₘₐₓ (maximum reaction velocity) values and the type of enzyme inhibition, distinguishing between competitive, noncompetitive and uncompetitive inhibitors (Stryer et al., 2002). The inhibition constant (Kᵢ) was determined by plotting the slopes of the Lineweaver-Burk plots against the inhibitor concentration.

### Molecular modelling

The program FUGUE (Shi et al., 2001) was used to identify possible structural motifs for the inhibitor peptide against a curated protein database, HOMSTRAD which contained 1034 protein families and 10230 aligned structures (Mizuguchi et al., 1998). FUGUE scans the database of structural profiles, calculates the sequence-structure compatibility scores and produces a list of potential homologues and alignments. Specificity, sensitivity and ranking are calculated according to the Z-score. Z-score thresholds of 6 indicate 99% specificity, and 5 indicate 95% specificity. A model of the inhibitor peptide was constructed using SYBYL /Tripos. Potential metal binding sites in the inhibitor peptide were identified by the locus of the metal atom positions relative to the backbone and C atom positions of the model peptide, using in-house software.

### Results

### Determination of P. gingivalis Arg- and Lys-specific proteolytic activity using chromogenic substrates

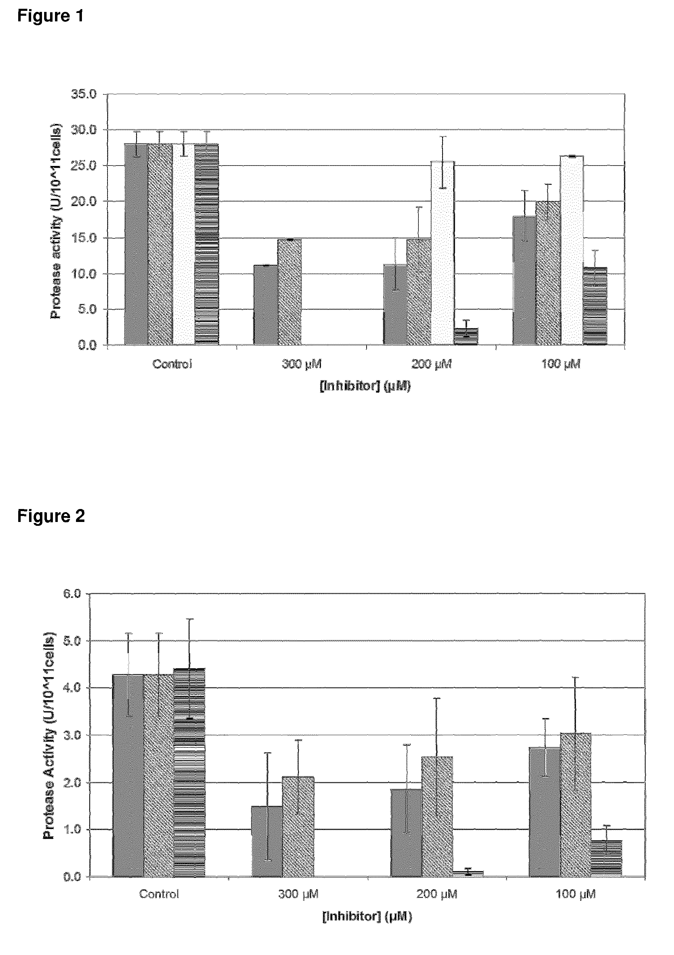

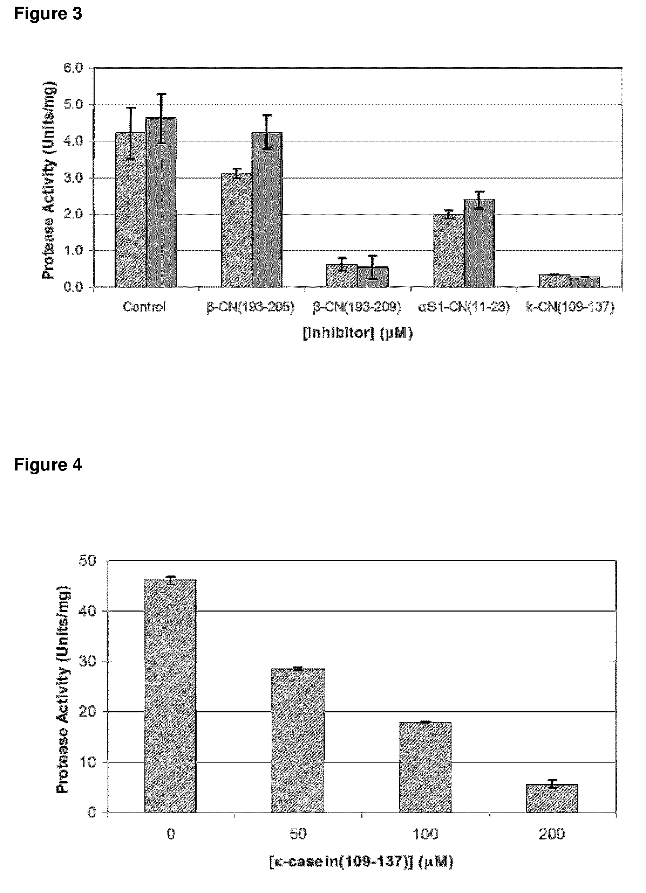

At 200 µM peptide concentration, κ-casein(109-137) showed the most efficacious results with ∼90% inhibition of whole cell Arg- and Lys-specific proteolytic activity while α_{S1}-casein(11-23) exhibited ∼70% and ∼60% inhibition of whole cell Arg- and Lys-specific proteolytic activity respectively (Figure 1, Figure 2 and Table 1, Table 2; see figure legends above under the "Brief description of the drawings" heading for the shading key). β-casein(193-209) inhibited 50% of both whole cell Arg- and Lys-specific proteolytic activity while the shorter β-casein peptide(193-205) inhibited only 15% Arg-specific proteolytic activity (Figure 1, Figure 2 and Table 1, Table 2; see figure legends above under the "Brief description of the drawings" heading for the shading key). The peptides were analysed at various concentrations and the % inhibition of proteolytic activity demonstrated a dose response to the peptide concentrations (Figure 1, Figure 2; see figure legends above under the "Brief description of the drawings" heading for the shading key). Subsequently, purified outer membrane protease complexes, comprising RgpA and Kgp, were used in the proteolytic assays to ensure that the proteolytic inhibition observed was not an artefact of other proteases likely present in whole cells. Accordingly, the peptides inhibited Arg- and Lys-specific proteolytic activity and even showed increased inhibitory potency with only 100 µM peptide (Figure 3, Table 1, Table 2; see figure legend above under the "Brief description of the drawings" heading for the shading key).

κ-casein(109-137), which exhibited the most significant inhibition of Arg- and Lys-specific proteolytic activity, was further assessed. The peptide was assessed against purified RgpB to determine the binding location of the peptide. Inhibition of RgpB proteolytic activity indicated that the peptide binds to the protease itself and not the protease complex (Figure 4, Table 1).

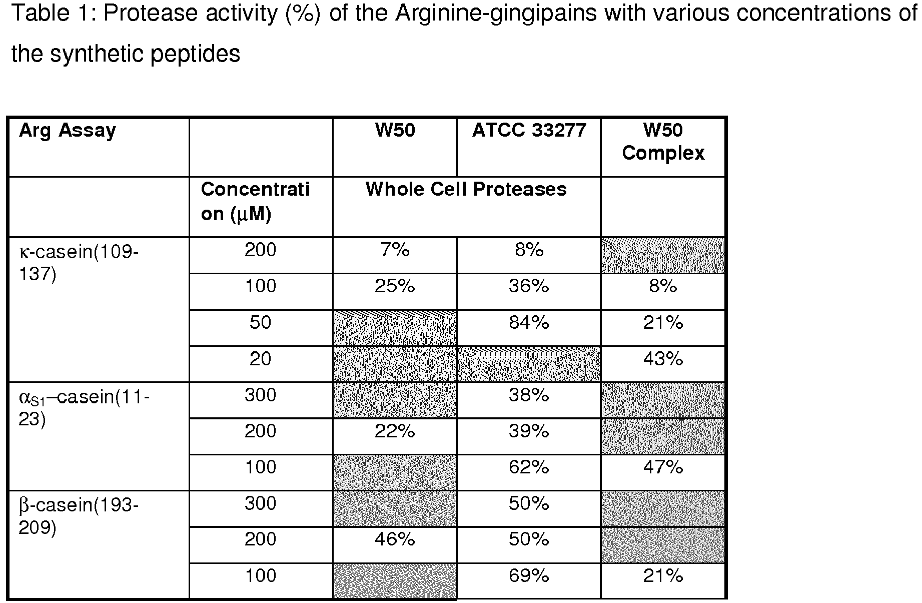

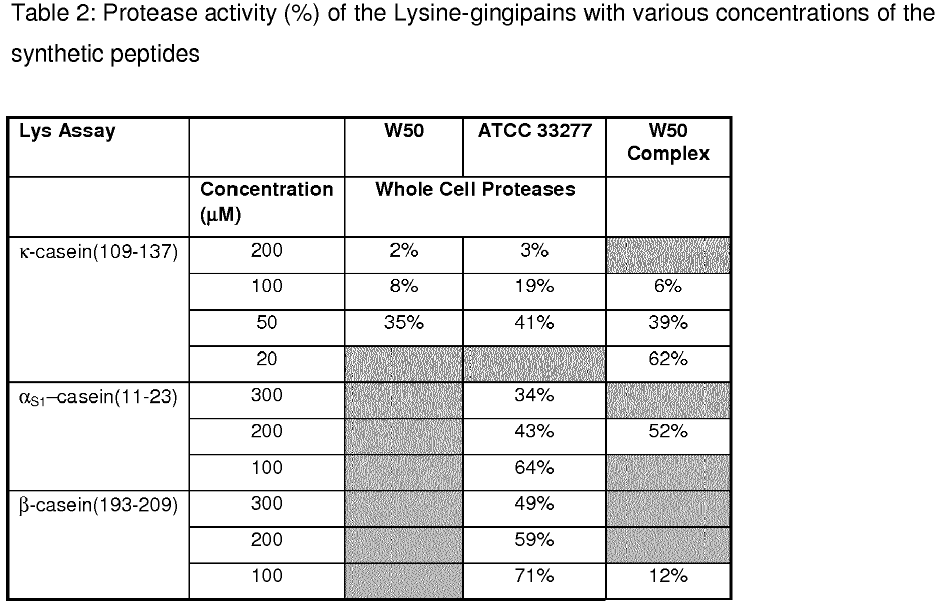

In order to delineate the residues essential for inhibition, a series of peptides were synthesized without the Lys-rich N-terminal region; κ-casein(117-123), κ-casein(127-137) and κ-casein(117-137). These synthetic peptides were screened for *P. gingivalis* proteinase inhibition in whole cell assays with fluorescent BSA and in proteolytic assays using purified RgpA-Kgp complexes with a chromogenic substrate (Table 3, Table 4, Figure 5). Only κ-casein(117-137) exhibited inhibitory activity with similar inhibitory potencies to the longer κ-casein(109-137). This demonstrated the lack of importance of the Lys residues in the N-terminal region for proteinase inhibition. Purified κ-casein(106-169) displayed similar efficacy to the shorter κ-casein(109-137) when tested in whole cell assays with fluorescent BSA (Figure 5).

A fluorescent substrate, DQ™ Green BSA was also used to measure the inhibitory activity of the casein peptides against whole *P. gingivalis* cell proteinase activity. Prior to the addition of the peptides, the assay was optimized for number of cells per well and incubation time. A 2 h incubation period with 10⁹ cells per well was selected for the assay as the rate of protein hydrolysis was linear under these conditions. The non-glycosylated κ-casein(106-169) peptide derived from casein, inhibited 60% of the proteinase activity against BSA at 500 µM peptide concentration in the assays. The synthetic shorter fragment κ-casein(106-137) had little effect on the Arg- and Lys-protease activity in chromogenic assays at 200 µM concentration while inhibiting ∼37% protease activity at 500 µM peptide concentration in the fluorescence assays (Table 4 and Figure 5).

**Table 3. Activity of the Arg- and Lys-specific proteinases from whole cells of P. gingivalis strain ATCC 33277, with various concentrations of the synthetic κ-casein peptides measured using chromogenic substrates (BApNA and GPK-NA)**

| | **Concentration (µM)** | **Arg-Specific Activity (Units/10¹¹ cells)** | **(%)** | **Lys-Specific Activity (Units/10¹¹ cells)** | **(%)** |
|---|---|---|---|---|---|
| Control | 0 | 17.99 ± 1.11 | 100% | 4.41 ± 1.07 | 100% |
| κ-casein(109-137) | 50 | 12.66 ± 2.83^{a,b} | 70% | 1.69 ± 0.64^{a} | 41% |
| | 100 | 6.89 ± 2.51^{a} | 38% | 0.76 ± 0.30^{a} | 19% |
| | 200 | 1.48 ± 1.18^{a} | 8% | 0.10 ± 0.06^{a} | 3% |
| κ-casein(117-123) | 200 | 20.04 ± 1.72^{e} | 111% | n/d | - |
| | 500 | 16.10 ± 0.57^{c,d} | 90% | n/d | - |
| | 1000 | 16.52 ± 3.26^{c,d} | 92% | n/d | - |
| κ-casein(127-137) | 200 | 18.26 ± 1.78^{d,e} | 102% | n/d | - |
| | 500 | 17.39 ± 0.33^{c,d} | 97% | n/d | - |
| | 1000 | 14.83 ± 1.19 ^{a,b,c} | 82% | n/d | - |

| | | | | | |
|---|---|---|---|---|---|
| *not determined ^{a}Significantly different (p<0.05) from the control ^{b,c,d,e}Significantly different (p <0.05) from other values not similarly marked | | | | | |

**Table 4. Activity of the purified RgpA-Kgp proteinase adhesin complexes of P. gingivalis W50 with various concentrations of the synthetic κ-casein peptides measured using chromogenic substrates (BApNA and GPK-NA)**

| | **Concentration (µM)** | **Arg-specific Activity (Units/mg)** | **(%)** | **Lys-specific Activity (Units/mg)** | **(%)** |
|---|---|---|---|---|---|
| Control | 0 | 4.13 ± 1.11^{d} | 100% | 4.67 ± 1.01 | 100% |
| κ-casein(109-137) | 20 | 1.80 ± 0.06^{a,c} | 43% | 2.90 ± 0.55^{a} | 62% |
| | 50 | 0.87 ± 0.38^{a,b} | 21% | 1.80 ± 0.91^{a} | 39% |
| | 100 | 0.33 ± 0.28^{a,b} | 8% | 0.28 ± 0.18^{a,b} | 6% |
| κ-casein(117-137) | 50 | 1.95 ± 0.38^{a,c} | 46% | 1.34 ± 0.25^{a,c} | 29% |
| | 100 | 0.41 ± 0.11^{a,b} | 10% | 0.98 ± 0.053^{a,c} | 21% |
| | 200 | 0.33 ± 0.02^{a,b} | 8% | 0.41 ± 0.014 ^{a,b} | 9% |
| κ-casein(117-123) | 200 | 3.68 ± 1.69^{d} | 89% | 4.62 ± 0.51 | 99% |
| κ-casein(127-137) | 200 | 4.49 ± 2.05^{d} | 109% | 3.86 ± 0.09^{a,d} | 83% |
| κ-casein(106-137) | 200 | 3.51 ± 1.77^{d} | 85% | 4.15 ± 0.64^{a,d} | 89% |

| | | | | | |
|---|---|---|---|---|---|
| *not determined ^{a}Significantly different (p < 0.05) from the control ^{b,c,d}Significantly different (p < 0.05) from other values not similarly marked. | | | | | |

### Zinc Chloride as a Co-inhibitor

ZnCl₂ is a potential co-inhibitor that has been shown to increase the inhibitory potency of several protease inhibitors such as benzamidine and chlorhexidine. To investigate the potential of zinc as a co-inhibitor, whole cell Arg- and Lys-specific protease assays were carried out with peptides [κ-casein(109-137)/ β-casein(193-209)/ α_{S1}-casein(11-23)] and ZnCl₂ in a 1:4 ratio.

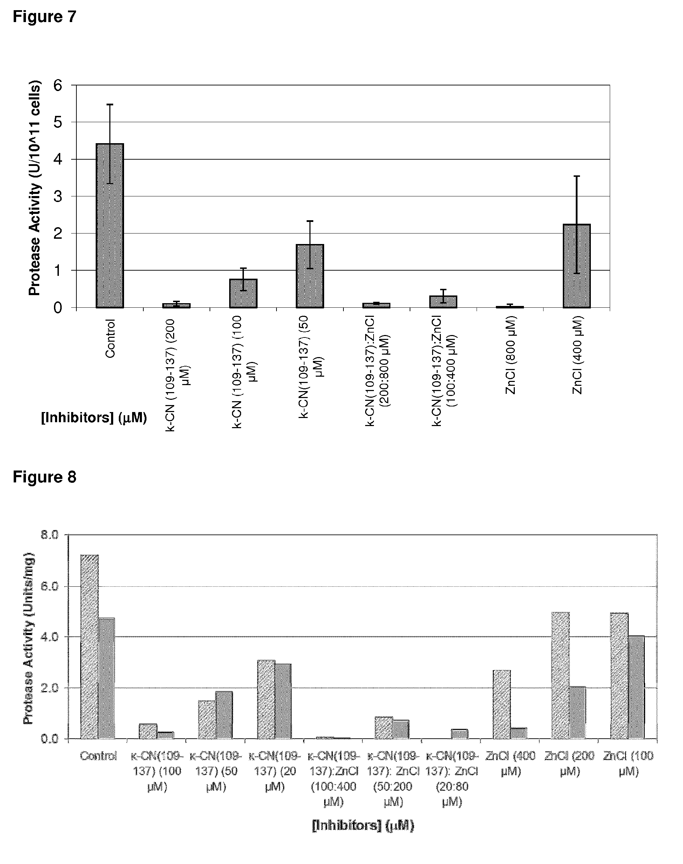

At 100 µM, κ-casein(109-137) inhibited whole cell Arg-specific proteolytic activity by 60%, while ZnCl₂ at 400 µM exhibited 30% inhibition. However, when κ-casein(109-137) and ZnCl₂ were combined in a 1:4 ratio, the inhibition increased to 90% (Figure 6). The peptide zinc mixtures also exhibited increased inhibition of Arg-specific proteolytic activity when analysed with purified protease complexes (Figure 8; see figure legend above under the "Brief description of the drawings" heading for the shading key). Similar results were observed in the Lys-specific protease assays with κ-casein(109-137): addition of ZnCl₂ increased inhibitory potency to 90% compared to the peptides potency when analysed individually (Figure 7). Based on the FIC index, the effect of Zn²⁺ is synergistic (Table 5).

**Table 5: Fractional Inhibitory constant indices to assess synergy of inhibition of the gingipains by κ-casein (109-137) peptide with or without Zn(II).**

| **Enzyme** | **Activity with ATCC33277^{a}** | | | | |
|---|---|---|---|---|---|
| | **No inhibitor** | **κ-casein (109-137) (100 µM)** | **Zinc(II) (400 µM)** | **κ-casein** + **Zinc(II) (100:400 µM)** | **FIC Index^{b}** |
| Arg-gingipains | 18.1 + 5.69 | 6.92 ± 2.51 | 12.1 ± 3.32 | 1.95 ± 1.29 | 0.44 |
| Lys-gingipains | 4.4 ± 1.07 | 0.76 ± 0.30 | 2.2 ± 1.31 | 0.31 ± 0.18 | 0.54 |

| | | | | | |
|---|---|---|---|---|---|
| a Activity ± standard deviation. b Fractional inhibitory concentration index | | | | | |

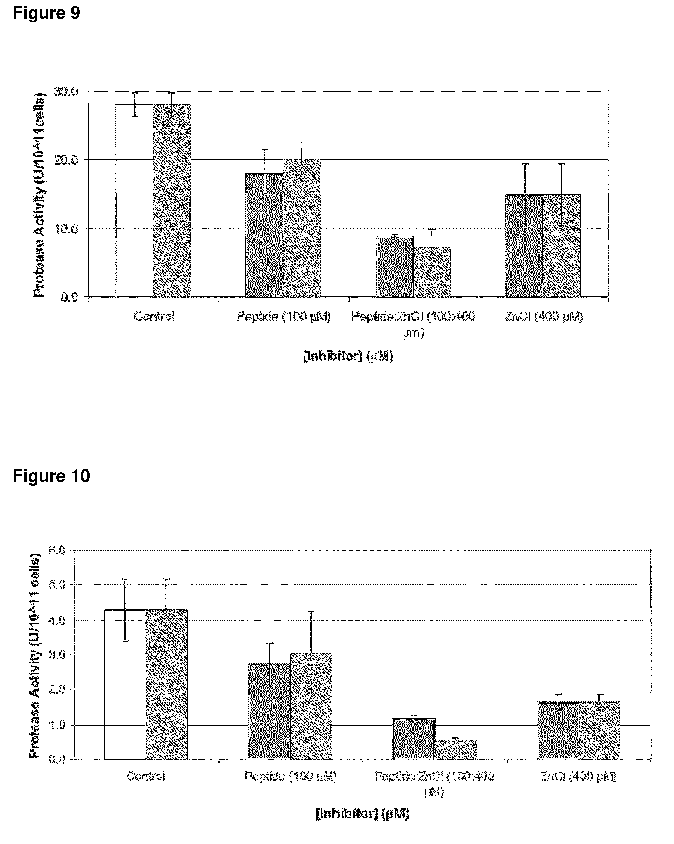

β-casein(193-209) at 100 µM inhibited Arg-specific proteolytic activity by 30%, while ZnCl₂ at 400 µM exhibited 50% inhibition. When combined in a 1:4 ratio, the proteolytic inhibition increased to 70% (Figure 9; see figure legend above under the "Brief description of the drawings" heading for the shading key). In the Lys-specific protease assays, β-casein(193-209): ZnCl₂ mixture increased inhibitory potency to 90% compared to the inhibitors' potency when analysed individually (Figure 10; see figure legend above under the "Brief description of the drawings" heading for the shading key). The FIC values of β-casein(193-209) and ZnCl₂ indicates that the inhibition is synergistic (Table 6).

**Table 6: Fractional Inhibitory constant indices to assess synergy of inhibition of the gingipains by β-casein (193-209) peptide with or without Zn(II).**

| **Enzyme** | **Activity with ATCC33277^{a}** | | | | |
|---|---|---|---|---|---|
| | **No inhibitor** | **β-casein (193-209) (100 µM)** | **Zinc(II) (400 µM)** | **β-casein** + **Zinc(II) (100:400 µM)** | **FIC Index^{b}** |
| Arg-gingipains | 29.23 + 0.06 | 20.03 ± 2.51 | 14.83 ± 4.60 | 7.23 ± 2.63 | 0.849 |
| Lys-gingipains | 4.28 ± 0.88 | 3.03 ± 1.20 | 1.65 ± 0.22 | 0.52 ± 0.1 | 0.487 |

| | | | | | |
|---|---|---|---|---|---|
| a Activity ± standard deviation. b Fractional inhibitory concentration index | | | | | |

α_{S1}-casein(11-23) inhibited Arg-specific proteolytic activity by 40% at 100 µM, while ZnCl₂ inhibited 50% Arg-specific proteolytic activity at 400 µM. Proteolytic inhibition increased to 70% when the inhibitors were combined in a 1:4 ratio (Figure 9; see figure legend above under the "Brief description of the drawings" heading for the shading key). In the Lys-specific protease assays, the inhibitor mixture increased inhibitory potency to 85% (Figure 10; see figure legends above under the "Brief description of the drawings" heading for the shading key). In contrast, the FIC values calculated for α_{S1}-casein(11-23) and ZnCl₂ indicates that the mixture of both inhibitors together is not synergistic but is additive (Table 7).

**Table 7: Fractional Inhibitory constant indices to assess synergy of inhibition of the gingipains by α_{S1}-casein (11-23) peptide with or without Zn(II).**

| **Enzyme** | **Activity with ATCC33277^{a}** | | | | |
|---|---|---|---|---|---|
| | **No inhibitor** | **α_{S1}-casein (11-23) (100 µM)** | **Zinc(II) (400 µM)** | **α_{S1}-casein** + **Zinc(II) (100:400 µM)** | **FIC Index^{b}** |
| Arg-gingipains | 29.23 + 0.06 | 18.00 ± 3.53 | 14.83 ± 4.60 | 8.89 ± 0.29 | 1.095 |
| Lys-gingipains | 4.28 ± 0.88 | 2.73 ± 0.60 | 1.65 ± 0.22 | 1.18 ± 0.1 | 1.147 |

| | | | | | |
|---|---|---|---|---|---|
| a Activity ± standard deviation. b Fractional inhibitory concentration index | | | | | |

### Determination of Inhibition Constant (Kᵢ) and Mechanism of Inhibition

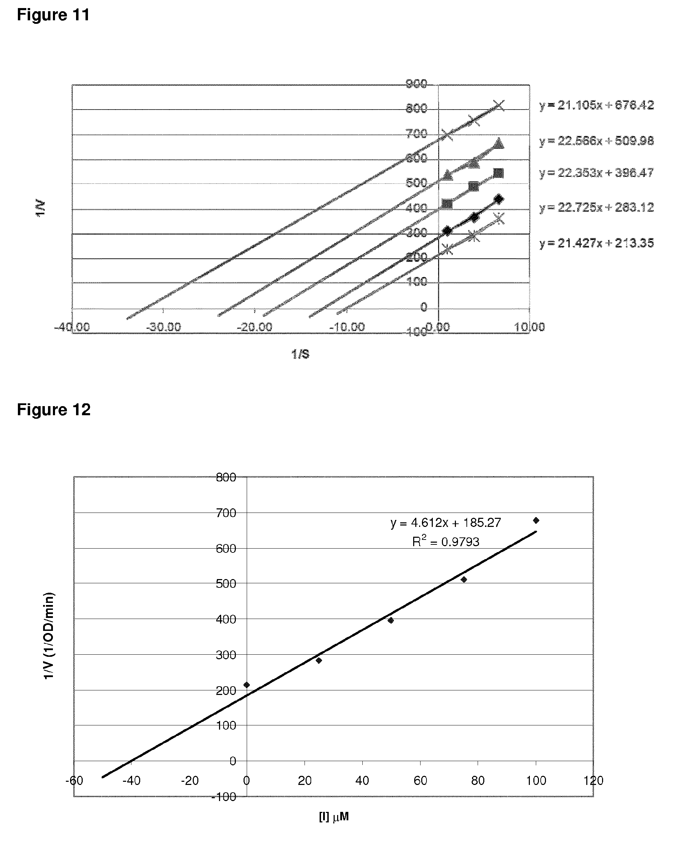

An enzyme kinetics assay was carried out with purified RgpB to determine the inhibition constant and the mechanism of inhibition of the κ-casein(109-137) peptide. The Lineweaver-Burk plot generated indicates uncompetitive inhibition by κ-casein(109-137) (Figure 11; see figure legend above under the "Brief description of the drawings" heading for the shading key). In the case of uncompetitive inhibition, the Kᵢ value is equal to the value of the inhibitor concentration required for 50% inhibition (IC₅₀). The Kᵢ value signifies the dissociation constant for binding of the inhibitor to the ES complex, and in this case it is 40.2 µM (Figure 12).

### BLAST Search and sequence alignment of the casein-derived peptides

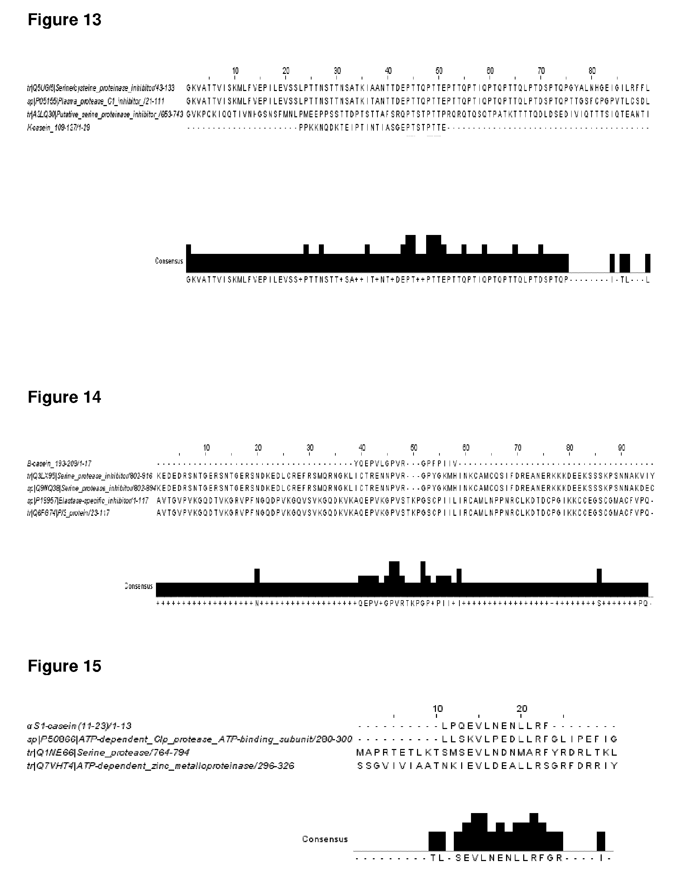

The α_{S1}-casein(11-23) sequence demonstrates sequence similarity to ATP- and substrate-binding ClpX and ClpA protease subunit (chaperone-like proteases) (Figure 15). ATP- and substrate-binding ClpX and ClpA are subunits of an ATP-dependent serine protease complex called ClpXP, ClpAP respectively, that is important for stress responses in microorganisms. In *Staphylococcus aureus,* ClpXP plays a role for survival in osmotic stress, oxidative stress and cold. In *P. gingivalis,* loss of ClpXP had no effect on oxidative stress tolerance but it is thought to be important for invasion of host epithelial cells, thermo-tolerance and biofilm formation. ClpAP and ClpXP do not possess identical activities but are similar. The casein sequence also showed similarity to an ATP-dependent zinc metalloprotease, FtsH 1, which also plays a role for biofilm formation in *P. gingivalis.* The casein sequence does not show any similarity to known protease inhibitors.

β-casein(193-209) shows sequence similarity to a few protease inhibitors (Figure 14). The Serine protease inhibitor Kazal-type 5 or LEKTI (Lympho-epithelial Kazal-type-related inhibitor) is a serine protease inhibitor that contains several distinct inhibitor units. The inhibitor is thought to be important for the anti-inflammatory and/or antimicrobial protection of mucous epithelia and has been shown to inhibit trypsin 1, cathepsin G, plasmin, subtilisin-A and elastase-2. Elafin or otherwise known as SKALP (Skin-derived antileukoprotease) is a serine protease inhibitor as well which inhibits elastase, arginyl peptidase, proteinase 3, and myeloblastin but does not inhibit trypsin, α-chymotrypsin, cathepsin G and plasmin. It mainly acts as a serine protease inhibitor but is also involved in anti-inflammatory functions. It also exhibits antimicrobial and antifungal properties against *Haemophilus influenza, Streptococcus pneumoniae, Aspergillus fumigatus,* and *Candida albicans,* albeit independent of its protease inhibitory function.

A BLAST search revealed sequence similarities of κ-casein(109-137) to a few protease inhibitors (Figure 13). Among them, plasma protease C1 inhibitor (Q5UGI5 and P05155) or Serpin G1 is believed to be potentially crucial in the regulation of vascular permeability and suppression of inflammation; inhibiting factor Xlla and plasma kallikrein (proteases of the plasma kallikrein-kinin system), C1r or C1s proteases (complement system), plasmin and tissue plasminogen activator (fibrinolytic system) and factor XI and thrombin (coagulation system) .

At 200 µM concentration, κ-casein(109-137) exhibited ∼93% inhibition of the Arg-specific proteolytic activity and 98% inhibition of the Lys-specific proteolytic activity in the whole cell assay. The peptide also inhibited purified RgpB protease by more than 80% at 200 µM concentrations (Table 1 and Table 2). These results are particularly significant as the peptide has 3 lysyl residues but no arginyl residues, therefore, it would not be cleaved by the RgpB protease.

α_{S1}-casein(11-23) inhibited whole cell protease activity by ∼60% while β-casein(193-209) showed ∼40-50% inhibition of both Arg- and Lys-specific proteolytic activity. The results also exhibit improved protease inhibitory function against the purified protease complexes. β-casein(193-205) however, was not as potent against both gingipains compared to the slightly longer peptide. This seems to indicate that the extra residues or the length of the peptide plays an important role in the peptide's inhibitory function.

ZnCl₂ is a potential co-inhibitor that has been shown to increase the inhibitory potency of several protease inhibitors such as benzamidine and chlorhexidine. The effects of different peptide to zinc ratios were investigated to determine if there is a synergistic effect between both inhibitors. An FIC index is used to calculate synergy. When a combination of inhibitors is additive, the FIC index will equal 1. When the inhibitors are antagonistic, more inhibitors are required to produce the same effect, and the sum will be more than 1, while a synergistic effect, when a combination of the two inhibitors is more effective than they are separately, will have a sum less than 1. Based on the FIC indices calculated from whole cell Arg- and Lys-specific protease assay results, the κ-casein(109-137) ZnCl₂ and β-casein(193-209) ZnCl₂ mixtures were synergistic while the α_{S1}-casein(11-23) ZnCl₂ mixture was additive (Table 5, 6 and 7).

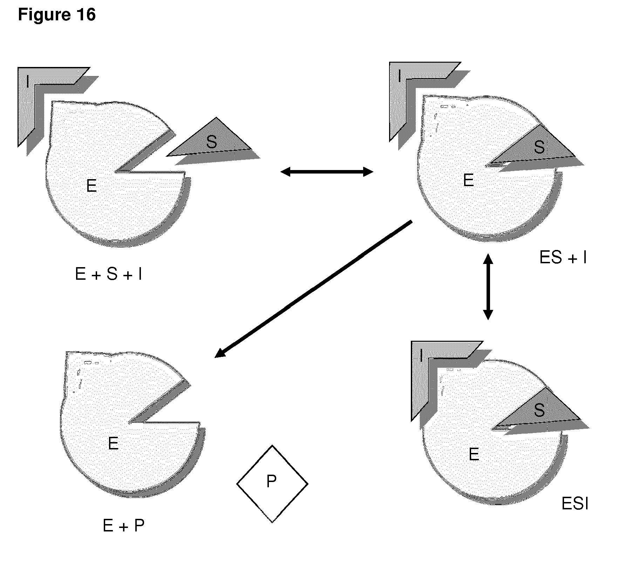

A Lineweaver-Burk analysis of the kinetics of gingipain activity enables the determination of type of enzyme inhibition, distinguishing between competitive, noncompetitive and uncompetitive inhibitors. Competitive inhibitors have the same y-intercept as the uninhibited enzyme (Vₘₐₓ is the same) but with different slopes and x-intercepts for each inhibitor concentration (different Kₘ). Noncompetitive inhibition produces plots with the same x-intercept as the uninhibited enzyme (K*ₘ* remains the same) but it has different slopes and *y*-intercepts (decreases Vₘₐₓ). Uncompetitive inhibition causes different intercepts on both the *y*- and *x*-axes but produces the same slopes. Based on the enzyme kinetics assay and the Lineweaver-Burk plots, the κ-casein (109-137) peptide is an uncompetitive inhibitor. Without being bound by any theory or mechanism of action, an uncompetitive inhibitor can mean that the peptide only binds to the enzyme-substrate complex preventing product formation. Both Kₘ and Vₘₐₓ values decreases. The binding of the substrate is believed to induce a conformational change in the enzyme. The new conformation enables the binding of the inhibitor (Figure 16). This differs from a non-competitive inhibitor, which can bind to both the enzyme and the enzyme-substrate complex, decreasing Vₘₐₓ (inhibitor hampers catalysis) but not changing Kₘ value (does not affect the substrate's affinity to the enzyme).

The inhibition constant of the peptide is 40.2 µM and in the case of uncompetitive inhibitors, the Kᵢ value is equal to the IC₅₀ value. The comparison of the Kᵢ value with other inhibitors of RgpB reveals that this peptide inhibitor has comparable Kᵢ values and is a moderate inhibitor of RgpB, sharing similar RgpB Kᵢ values to chlorhexidine (2.62 x10⁻⁴M) and doxycycline, an uncompetitive inhibitor, has an IC₅₀ against RgpB of 3 µM.

A BLAST search revealed sequence similarities of α_{S1}-casein(11-23) to ATP- and substrate-binding ClpX and ClpA protease subunit (chaperone-like proteases) and to an

ATP-dependent zinc metalloprotease, FtsH 1, which plays a role for biofilm formation in *P. gingivalis.* β-casein(193-209) sequence demonstrates sequence similarity to a few protease inhibitors such as serine protease inhibitor Kazal-type 5 or LEKTI (Lympho-epithelial Kazal-type-related inhibitor), and elafin. Both these serine protease inhibitors also exhibit antimicrobial functions independent of its protease inhibitory function. κ-casein(109-137) too shows partial sequence similarity to a few protease inhibitors. Among them are plasma protease C1 inhibitor (Q5UGI5 and P05155) or Serpin G1.

The program Fugue was used to identify possible structure motifs for the peptide against a curated protein database HOMSTRAD. A Z-score of 3.87 was obtained for κ-casein(109-137) peptide indicating 90% confidence for an α-helical structure. The possible structural motifs for κ-casein(109-137) include an α-helix spanning residues 109-126, a turn, and another α-helix spanning residues 129-137. Based on these motifs, a model of the κ-casein(109-137) peptide was constructed using the SYBYL software. A model of the κ-casein(109-137) inhibiting RgpB was developed based on the crystal structure of the RgpB (PDB 1cvr) and the proposed model of κ-casein(109-137) with the potential metal binding sites. Figure 17 (a) shows the proposed model highlighting the residues of RgpB active site involved in interacting with the peptide inhibitor, κ-casein(109-137), Zn(II) and the synthetic substrate (BApNA). This model highlights the electrostatic interactions between residues His²¹¹ and Glu¹⁵² of RgpB and residues Asp¹¹⁵ and Glu¹¹⁸ of κ-casein(109-137) with Zn(II). Residue Ile¹²² of κ-casein(109-137) forms a hydrophobic interaction with the substrate BApNA. Residues _{T}rp²⁸⁴ and Cys²⁴⁴ of RgpB also interact with the Arg residue and the amide bond respectively, of the BApNA substrate. Figure 17 (b) shows the proposed molecular model of κ-casein(109-137) binding to the enzyme-substrate complex (RgpB-BApNA) in the presence of Zn(II). This model is consistent with the experimental evidence of synergistic inhibition.

### Compositions and Formulations

To help illustrate compositions embodying aspects of the invention directed to treatment or prevention, the following sample formulations are provided.

The following is an example of a toothpaste formulation.

| Ingredient | % w/w |
|---|---|
| Dicalcium phosphate dihydrate | 50.0 |
| Glycerol | 20.0 |
| Sodium carboxymethyl cellulose | 1.0 |
| Sodium lauryl sulphate | 1.5 |
| Sodium lauroyl sarconisate | 0.5 |
| Flavour | 1.0 |
| Sodium saccharin | 0.1 |
| Chlorhexidine gluconate | 0.01 |
| Dextranase | 0.01 |
| Compound, peptide or peptidomimetic of the invention | 0.2 |
| Water | balance |

The following is an example of a further toothpaste formulation.

| Ingredient | % w/w |
|---|---|
| Dicalcium phosphate dihydrate | 50.0 |
| Sorbitol | 10.0 |
| Glycerol | 10.0 |
| Sodium carboxymethyl cellulose | 1.0 |
| Lauroyl diethanolamide | 1.0 |
| Sucrose monolaurate | 2.0 |
| Flavour | 1.0 |
| Sodium saccharin | 0.1 |
| Sodium monofluorophosphate | 0.3 |
| Chlorhexidine gluconate | 0.01 |
| Dextranase | 0.01 |
| Compound, peptide or peptidomimetic of the invention | 0.1 |
| Water | balance |

The following is an example of a further toothpaste formulation.

| Ingredient | % w/w |
|---|---|
| Sorbitol | 22.0 |
| Irish moss | 1.0 |
| Sodium Hydroxide (50%) | 1.0 |
| Gantrez | 19.0 |
| Water (deionised) | 2.69 |
| Sodium Monofluorophosphate | 0.76 |
| Sodium saccharine | 0.3 |
| Pyrophosphate | 2.0 |
| Hydrated alumina | 48.0 |
| Flavour oil | 0.95 |
| Compound, peptide or peptidomimetic of the invention | 0.3 |
| sodium lauryl sulphate | 2.00 |

The following is an example of a liquid toothpaste formulation.

| Ingredient | % w/w |
|---|---|
| Sodium polyacrylate | 50.0 |
| Sorbitol | 10.0 |
| Glycerol | 20.0 |
| Flavour | 1.0 |
| Sodium saccharin | 0.1 |
| Sodium monofluorophosphate | 0.3 |
| Chlorhexidine gluconate | 0.01 |
| Ethanol | 3.0 |
| Compound, peptide or peptidomimetic of the invention | 0.2 |
| Linolic acid | 0.05 |
| Water | balance |

The following is an example of a mouthwash formulation.

| Ingredient | % w/w |
|---|---|
| Ethanol | 20.0 |
| Flavour | 1.0 |
| Sodium saccharin | 0.1 |
| Sodium monofluorophosphate | 0.3 |
| Chlorhexidine gluconate | 0.01 |
| Lauroyl diethanolamide | 0.3 |
| Compound, peptide or peptidomimetic of the invention | 0.2 |
| Water | balance |

The following is an example of a further mouthwash formulation.

| Ingredient | % w/w |
|---|---|
| Gantrez® S-97 | 2.5 |
| Glycerine | 10.0 |
| Flavour oil | 0.4 |
| Sodium monofluorophosphate | 0.05 |
| Chlorhexidine gluconate | 0.01 |
| Lauroyl diethanolamide | 0.2 |
| Compound, peptide or peptidomimetic of the invention | 0.3 |
| Water | balance |

The following is an example of a lozenge formulation.

| Ingredient | % w/w |
|---|---|
| Sugar | 75-80 |
| Corn syrup | 1-20 |
| Flavour oil | 1-2 |
| NaF | 0.01-0.05 |
| Compound, peptide or peptidomimetic of the invention | 0.3 |
| Mg stearate | 1-5 |
| Water | balance |

The following is an example of a gingival massage cream formulation.

| Ingredient | % w/w |
|---|---|
| White petrolatum | 8.0 |
| Propylene glycol | 4.0 |
| Stearyl alcohol | 8.0 |
| Polyethylene Glycol 4000 | 25.0 |
| Polyethylene Glycol 400 | 37.0 |
| Sucrose monostearate | 0.5 |
| Chlorhexidine gluconate | 0.1 |
| Compound, peptide or peptidomimetic of the invention | 0.3 |
| Water | balance |

The following is an example of a periodontal gel formulation.

| Ingredient | % w/w |
|---|---|
| Pluronic F127 (from BASF) | 20.0 |
| Stearyl alcohol | 8.0 |
| Compound, peptide or peptidomimetic of the invention | 3.0 |
| Colloidal silicon dioxide (such as Aerosil® 200™) | 1.0 |
| Chlorhexidine gluconate | 0.1 |
| Water | balance |

The following is an example of a chewing gum formulation.

| Ingredient | % w/w |
|---|---|
| Gum base | 30.0 |
| Calcium carbonate | 2.0 |
| Crystalline sorbitol | 53.0 |
| Glycerine | 0.5 |
| Flavour oil | 0.1 |
| Compound, peptide or peptidomimetic of the invention | 0.3 |
| Water | balance |

It should be understood that while the invention has been described in detail herein, the examples are for illustrative purposes only. Other modifications of the embodiments of the present invention that are obvious to those skilled in the art of molecular biology, dental treatment, and related disciplines are intended to be within the scope of the invention.

### References

Berenbaum, M. C. (1978). "A method for testing for synergy with any number of agents." J Infect Dis 137(2): 122-30.
Chen, Y.-Y., K. J. Cross, R. A. Paolini, J. E. Fielding, N. Slakeski, and E. C. Reynolds. 2002. CPG70 is a novel basic metallocarboxypeptidase with C-terminal polycystic kidney disease domains from Porphyromonas gingivalis. J. Biol. Chem. 277:23433-23440.
Fletcher, H. M., H. A. Schenkein, and F. L. Macrina. 1994. Cloning and characterization of a new protease gene (prtH) from Porphyromonas gingivalis. Infect. Immun. 62:4279-4286.
Grenier, D., S. Imbeault, P. Plamondon, G. Grenier, K. Nakayama, and D. Mayrand. 2001. Role of gingipains in growth of Porphyromonas gingivalis in the presence of human serum albumin. Infect. Immun. 69:5166-5172.
Malkoski, M., S. G. Dashper, N. O'Brien-Simpson, G. H. Talbo, M. Macris, K. J. Cross, and E. C. Reynolds. 2001. Kappacin, a novel antibacterial peptide from bovine milk. Antimicrob. Agents Chemother. 45:2309-2315.
Mizuguchi, K., C. M. Deane, et al. (1998). "HOMSTRAD: a database of protein structure alignments for homologous families." Protein Sci 7(11): 2469-71.
O'Brien-Simpson, N. M., S. G. Dashper, et al. (1998). "Histatin 5 is a substrate and not an inhibitor of the Arg- and Lys-specific proteinases of Porphyromonas gingivalis." Biochem Biophys Res Commun 250(2): 474-8.
O'Brien-Simpson, N. M., R. A. Paolini, et al. (2001). "Role of RgpA, RgpB, and Kgp proteinases in virulence of Porphyromonas gingivalis W50 in a murine lesion model." Infect Immun 69(12): 7527-34.
Pathirana, R. D., N. M. O'Brien-Simpson, et al. (2006). "Characterization of proteinase-adhesin complexes of Porphyromonas gingivalis." Microbiology 152(Pt 8): 2381-94.
Pike, R., W. McGraw, J. Potempa, and J. Travis. 1994. Lysine- and arginine-specific proteinases from Porphyromonas gingivalis. Isolation, characterization, and evidence for the existence of complexes with hemagglutinins. J. Biol. Chem. 269:406-411.
Shi, J., T. L. Blundell, et al. (2001). "FUGUE: sequence-structure homology recognition using environment-specific substitution tables and structure-dependent gap penalties." J Mol Biol 310(1): 243-57.
Stryer, L., J. M. Berg, et al. (2002). Biochemistry Fifth Edition. New York, W.H. Freeman and Company.
Yoshioka, M., D. Grenier, and D. Mayrand. 2003. Monitoring the uptake of protein- derived peptides by Porphyromonas gingivalis with fluorophore-labeled substrates. Curr. Microbiol. 47:1-4.

## Claims

1. A peptide or peptidomimetic thereof for inhibiting, reducing or preventing the activity of a gingipain, wherein the peptide or peptidomimetic thereof consists of an amino acid sequence selected from SEQ ID NO: 3 or SEQ ID NO: 5, or a variant thereof, wherein said variant comprises conservative substitutions therein and wherein said variant is at least 80% identical to SEQ ID NO 3 or 5.

2. A peptide or peptidomimetic thereof according to claim 1, wherein the amino acid sequence is SEQ ID NO: 3.

3. A peptide or peptidomimetic thereof according to claim 1, wherein the amino acid sequence is SEQ ID NO: 5.

4. A peptide or peptidomimetic thereof according to claim 1, wherein said variant is at least 90% identical to SEQ ID NO: 3 or 5.

5. A peptide or peptidomimetic thereof according to claim 1, wherein the variant consists of an amino acid sequence selected from the group consisting of SEQ ID NO 10 to 32.

6. A peptide or peptidomimetic thereof according to claim 1, wherein the gingipain is from *Porphyromonas gingivalis.*

7. A peptide or peptidomimetic thereof according to any preceding claim for use in the treatment of periodontal disease or for use in the treatment of or in reducing the risk of developing other diseases, conditions or syndromes that are a consequence of or associated with periodontal disease.

8. A peptide or peptidomimetic thereof according to claim 7, wherein the disease associated with periodontal disease is cardiovascular disease.

9. A pharmaceutical composition for inhibiting, reducing or preventing the activity of a gingipain, comprising a protein or peptidomimetic thereof of any one of claims 1 to 6 and a pharmaceutically acceptable carrier.

10. A pharmaceutical composition according to claim 9, wherein said pharmaceutical composition further comprises a divalent cation.

11. A pharmaceutical composition according to claim 10, wherein the divalent cation is zinc.

12. A pharmaceutical composition according to any one of claims 9 to 11, for use in the treatment of periodontal disease or for use in the treatment of or in reducing the risk of developing other diseases, conditions or syndromes that are a consequence of or associated with periodontal disease.

13. A pharmaceutical composition according to claim 12, wherein the disease associated with periodontal disease is cardiovascular disease

14. An *in vitro* method of inhibiting a bacterial enzyme, wherein the enzyme is gingipain, comprising contacting the enzyme with a peptide or peptidomimetic thereof according to any one of claims 1 to 6.


SEQUENCE LISTING

<110>  Oral Health Australia Pty Ltd

<120>  Protease inhibitory peptides

<130>  PC926339epa

<150>  AU 2009902841
<151>  2009-06-19

<160>  32

<170>  PatentIn version 3.5

<210>  1
<211>  13
<212>  PRT
<213>  Bos taurus

<400>  1

Leu Pro Gln Glu Val Leu Asn Glu Asn Leu Leu Arg Phe
1               5                   10

<210>  2
<211>  17
<212>  PRT
<213>  Bos taurus

<400>  2

Tyr Gln Glu Pro Val Leu Gly Pro Val Arg Gly Pro Phe Pro Ile Ile
1               5                   10                  15

Val

<210>  3
<211>  29
<212>  PRT
<213>  Bos taurus

<400>  3

Pro Pro Lys Lys Asn Gln Asp Lys Thr Glu Ile Pro Thr Ile Asn Thr
1               5                   10                  15

Ile Ala Ser Gly Glu Pro Thr Ser Thr Pro Thr Thr Glu
            20                  25

<210>  4
<211>  13
<212>  PRT
<213>  Bos taurus

<400>  4

Tyr Gln Glu Pro Val Leu Gly Pro Val Arg Gly Pro Phe
1               5                   10

<210>  5
<211>  21
<212>  PRT
<213>  Bos taurus

<400>  5

Thr Glu Ile Pro Thr Ile Asn Thr Ile Ala Ser Gly Glu Pro Thr Ser
1               5                   10                  15

Thr Pro Thr Thr Glu
            20

<210>  6
<211>  13
<212>  PRT
<213>  Bubalus bubalis

<400>  6

Leu Pro Gln Gly Val Leu Asn Glu Asn Leu Leu Arg Phe
1               5                   10

<210>  7
<211>  13
<212>  PRT
<213>  Capra hircus

<400>  7

Leu Ser Pro Glu Val Leu Asn Glu Asn Leu Leu Arg Phe
1               5                   10

<210>  8
<211>  13
<212>  PRT
<213>  Ovis aries

<400>  8

Leu Ser Ser Glu Val Leu Asn Glu Asn Leu Leu Arg Phe
1               5                   10

<210>  9
<211>  17
<212>  PRT
<213>  Capra hircus

<400>  9

Tyr Gln Glu Pro Val Leu Gly Pro Val Arg Gly Pro Phe Pro Ile Leu
1               5                   10                  15

Val

<210>  10
<211>  29
<212>  PRT
<213>  Syncerus caffer nanus

<400>  10

Pro Pro Lys Lys Asn Gln Asp Lys Thr Glu Ile Pro Thr Ile Asn Thr
1               5                   10                  15

Ile Ala Ser Gly Glu Pro Thr Ser Thr Pro Thr Ile Glu
            20                  25

<210>  11
<211>  29
<212>  PRT
<213>  Bos indicus

<400>  11

Pro Pro Lys Lys Asn Gln Asp Lys Thr Glu Ile Pro Thr Ile Asn Thr
1               5                   10                  15

Ile Ala Ser Gly Glu Pro Thr Ser Thr Pro Ile Thr Glu
            20                  25

<210>  12
<211>  29
<212>  PRT
<213>  Bos taurus

<220>
<221>  MISC_FEATURE
<222>  (25)..(25)
<223>  Xaa is glycosylated threonine

<400>  12

Pro Pro Lys Lys Asn Gln Asp Lys Thr Glu Ile Pro Thr Ile Asn Thr
1               5                   10                  15

Ile Ala Ser Gly Glu Pro Thr Ser Xaa Pro Thr Thr Glu
            20                  25

<210>  13
<211>  29
<212>  PRT
<213>  Bos taurus

<220>
<221>  MISC_FEATURE

<222>  (23)..(23)
<223>  Xaa is glycosylated threonine

<400>  13

Pro Pro Lys Lys Asn Gln Asp Lys Thr Glu Ile Pro Thr Ile Asn Thr
1               5                   10                  15

Ile Ala Ser Gly Glu Pro Xaa Ser Thr Pro Thr Thr Glu
            20                  25

<210>  14
<211>  29
<212>  PRT
<213>  Bos taurus

<220>
<221>  MISC_FEATURE
<222>  (28)..(28)
<223>  Xaa is glycosylated threonine

<400>  14

Pro Pro Lys Lys Asn Gln Asp Lys Thr Glu Ile Pro Thr Ile Asn Thr
1               5                   10                  15

Ile Ala Ser Gly Glu Pro Thr Ser Thr Pro Thr Xaa Glu
            20                  25

<210>  15
<211>  29
<212>  PRT
<213>  Bos taurus

<220>
<221>  MISC_FEATURE
<222>  (13)..(13)
<223>  Xaa is glycosylated threonine

<400>  15

Pro Pro Lys Lys Asn Gln Asp Lys Thr Glu Ile Pro Xaa Ile Asn Thr
1               5                   10                  15

Ile Ala Ser Gly Glu Pro Thr Ser Thr Pro Thr Thr Glu
            20                  25

<210>  16
<211>  29
<212>  PRT
<213>  Bos taurus

<220>
<221>  MISC_FEATURE

<222>  (19)..(19)
<223>  Xaa is phosphorylated serine

<400>  16

Pro Pro Lys Lys Asn Gln Asp Lys Thr Glu Ile Pro Thr Ile Asn Thr
1               5                   10                  15

Ile Ala Xaa Gly Glu Pro Thr Ser Thr Pro Thr Thr Glu
            20                  25

<210>  17
<211>  29
<212>  PRT
<213>  Bubalus bubalis

<400>  17

Pro Pro Lys Lys Asn Gln Asp Lys Thr Glu Ile Pro Thr Ile Asn Thr
1               5                   10                  15

Ile Val Ser Gly Glu Pro Thr Ser Thr Pro Thr Thr Glu
            20                  25

<210>  18
<211>  29
<212>  PRT
<213>  Bubalus bubalis

<400>  18

Pro Pro Lys Lys Asn Gln Asp Lys Thr Glu Ile Pro Thr Ile Asn Thr
1               5                   10                  15

Ile Ala Ser Val Glu Pro Thr Ser Thr Pro Thr Thr Glu
            20                  25

<210>  19
<211>  29
<212>  PRT
<213>  Oreamnos americanus

<400>  19

Pro Pro Lys Lys Asp Gln Asp Lys Thr Glu Ile Pro Thr Ile Asn Thr
1               5                   10                  15

Ile Ala Ser Gly Glu Pro Thr Ser Thr Pro Thr Thr Glu
            20                  25

<210>  20
<211>  29
<212>  PRT
<213>  Oreamnos americanus

<400>  20

Pro Pro Lys Lys Asn Gln Asp Lys Thr Glu Ile Pro Thr Ile Asn Thr
1               5                   10                  15

Ile Ala Ser Ala Glu Pro Thr Ser Thr Pro Thr Thr Glu
            20                  25

<210>  21
<211>  29
<212>  PRT
<213>  Capra hircus

<400>  21

Pro Pro Lys Lys Asp Gln Asp Lys Thr Glu Val Pro Ala Ile Asn Thr
1               5                   10                  15

Ile Ala Ser Ala Glu Pro Thr Ser Thr Pro Thr Thr Glu
            20                  25

<210>  22
<211>  21
<212>  PRT
<213>  Syncerus caffer nanus

<400>  22

Thr Glu Ile Pro Thr Ile Asn Thr Ile Ala Ser Gly Glu Pro Thr Ser
1               5                   10                  15

Thr Pro Thr Ile Glu
            20

<210>  23
<211>  21
<212>  PRT
<213>  Bos indicus

<400>  23

Thr Glu Ile Pro Thr Ile Asn Thr Ile Ala Ser Gly Glu Pro Thr Ser
1               5                   10                  15

Thr Pro Ile Thr Glu
            20

<210>  24
<211>  21
<212>  PRT
<213>  Bos taurus

<220>
<221>  MISC_FEATURE

<222>  (17)..(17)
<223>  Xaa is glycosylated threonine

<400>  24

Thr Glu Ile Pro Thr Ile Asn Thr Ile Ala Ser Gly Glu Pro Thr Ser
1               5                   10                  15

Xaa Pro Thr Thr Glu
            20

<210>  25
<211>  21
<212>  PRT
<213>  Bos taurus

<220>
<221>  MISC_FEATURE
<222>  (15)..(15)
<223>  Xaa is glycosylated threonine

<400>  25

Thr Glu Ile Pro Thr Ile Asn Thr Ile Ala Ser Gly Glu Pro Xaa Ser
1               5                   10                  15

Thr Pro Thr Thr Glu
            20

<210>  26
<211>  21
<212>  PRT
<213>  Bos taurus

<220>
<221>  MISC_FEATURE
<222>  (20)..(20)
<223>  Xaa is glycosylated threonine

<400>  26

Thr Glu Ile Pro Thr Ile Asn Thr Ile Ala Ser Gly Glu Pro Thr Ser
1               5                   10                  15

Thr Pro Thr Xaa Glu
            20

<210>  27
<211>  21
<212>  PRT
<213>  Bos taurus

<220>
<221>  MISC_FEATURE

<222>  (5)..(5)
<223>  Xaa is glycosylated threonine

<400>  27

Thr Glu Ile Pro Xaa Ile Asn Thr Ile Ala Ser Gly Glu Pro Thr Ser
1               5                   10                  15

Thr Pro Thr Thr Glu
            20

<210>  28
<211>  21
<212>  PRT
<213>  Bos taurus

<220>
<221>  MISC_FEATURE
<222>  (11)..(11)
<223>  Xaa is phosphorylated serine

<400>  28

Thr Glu Ile Pro Thr Ile Asn Thr Ile Ala Xaa Gly Glu Pro Thr Ser
1               5                   10                  15

Thr Pro Thr Thr Glu
            20

<210>  29
<211>  21
<212>  PRT
<213>  Bubalus bubalis

<400>  29

Thr Glu Ile Pro Thr Ile Asn Thr Ile Val Ser Gly Glu Pro Thr Ser
1               5                   10                  15

Thr Pro Thr Thr Glu
            20

<210>  30
<211>  21
<212>  PRT
<213>  Bubalus bubalis

<400>  30

Thr Glu Ile Pro Thr Ile Asn Thr Ile Ala Ser Val Glu Pro Thr Ser
1               5                   10                  15

Thr Pro Thr Thr Glu
            20

<210>  31
<211>  21
<212>  PRT
<213>  Oreamnos americanus

<400>  31

Thr Glu Ile Pro Thr Ile Asn Thr Ile Ala Ser Ala Glu Pro Thr Ser
1               5                   10                  15

Thr Pro Thr Thr Glu
            20

<210>  32
<211>  21
<212>  PRT
<213>  Capra hircus

<400>  32

Thr Glu Val Pro Ala Ile Asn Thr Ile Ala Ser Ala Glu Pro Thr Ser
1               5                   10                  15

Thr Pro Thr Thr Glu
            20